Europäisches Patentamt

European Patent Office

Office européen des brevets

(11) Publication number: **0 246 909 B1**

# EUROPEAN PATENT SPECIFICATION

(45) Date of publication of patent specification: **29.09.93**   (51) Int. Cl.⁵: **C12N  15/74**, C07K 15/00, A23C 19/032

(21) Application number: **87304557.9**

(22) Date of filing: **21.05.87**

---

(54) **Plasmids conferring phage insensitivity on bacteria.**

---

(30) Priority: **21.05.86 IE 1352/86**

(43) Date of publication of application:
**25.11.87 Bulletin  87/48**

(45) Publication of the grant of the patent:
**29.09.93 Bulletin  93/39**

(84) Designated Contracting States:
**AT BE CH DE ES FR GB GR IT LI LU NL SE**

(56) References cited:
**EP-A- 0 218 230**

**APPLIED AND ENVIRONMENTAL MICROBIOLOGY, vol. 50, no. 4, October 1985, pages 851-858, American Society for Microbiology, Washington, D.C., US; L.R. STEENSON et al.: "Streptococcus cremoris M12R transconjugants carrying the conjugal plasmid pTR2030 are insensitive to attack by lytic bacteriophages"**

(73) Proprietor: **THE NATIONAL DEVELOPMENT CORPORATION LIMITED**
**Haddington Court Haddington Road Dublin 4(IE)**

(72) Inventor: **Daly, Charles**
**39 Allendale Avenue**
**Bishopstown Cork(IE)**
Inventor: **Fitzgerald, Gerald F.**
**56 Halldene Grove**
**Bishopstown Cork(IE)**
Inventor: **Coffey Aidanof Ballylaneen**
**Kilmacthoma Co**
**Waterford(IE)**
Inventor: **Baumgartner, Andreas**
**Wiesenstrasse 47**
**CH-3010 Bern(CH)**
Inventor: **Costello, VeronicaA.**
**2 Cahill's Park**
**Tralee Co. Kerry(IE)**

(74) Representative: **Hildyard, Edward Martin et al**
**Frank B. Dehn & Co. Imperial House 15-19 Kingsway**
**London WC2B 6UZ (GB)**

---

EP 0 246 909 B1

FEMS MICROBIOLOGY LETTERS, vol. 35, 17th July 1986, pages 233-237, Elsevier, Amsterdam, NL; A. BAUMGARTNER et al.: "Conjugative co-transfer of lactose and bacteriophage resistance plasmids from Streptococcus cremoris UC653"

CHEMIE MIKROBIOLOGIE TECHNOLOGIE DER LEBENSMITTEL, vol. 10, 1986, pages 86-89, Freising-Weihenstephan, DE; A. WETZEL et al.: "Transfer of plasmid-mediated phage resistance in lactic acid streptococci"

## Description

This invention relates to plasmids which confer phage insensitivity on bacteria, particularly lactic acid bacteria, bacteria to which such plasmids have been introduced and use of such plasmids in the food industry, particularly the dairy industry.

It is known that the activity of a starter culture in dairy fermentations such as the production of cheese, cultured buttermilk, cultured butter or sour cream can be adversely affected by bacteriophage attack. It is accepted that there is an urgent need to extend the range of commercially stable phage-insensitive strains available to the dairy industry. Genetic studies on the lactic streptococci have suggested that genetic manipulation may be used to construct strains with new and/or improved industrial traits.

The terms "phage insensitivity" and "phage insensitive" are used throughout this application to denote an inability of a bacteriophage to propagate on a host strain. The term "resistance" is commonly used in the lactic streptococcal/bacteriophage literature to described hosts which do not allow phage propagation even though the "resistance" mechanism is not known, although in a strict scientific sense the term "resistance" implies an inability of a phage to adsorb to a host.

A number of plasmid-encoded mechanisms involved in host-mediated insensitivity to phage attack in the lactic streptococci have been described in the literature. The insensitivity may be at the level of adsorption, restriction-modification (R-M) of phage DNA, or by powerful, but as yet uncharacterized, mechanisms that prevent phage maturation.

Two plasmid-encoded adsorption blocking systems have been described by Sanders and Klaenhammer (19) and de Vos et al (20). These molecules, pME0030 (30 Mdal, from S. lactis ME2, ref. 19) and pSK112 (34 Mdal, from S. cremoris SK11, ref. 20) have the effect of significantly inhibiting phage adsorption to plasmid-containing hosts with a consequent suppression of phage replication. Elimination of the plasmids from their respective hosts results in highly efficient phage adsorption and an increased sensitivity to specific phage. However, since neither plasmid was reported to be conjugative, it may be difficult to exploit them to construct new phage insensitive starter culture strains.

Plasmids encoding R-M activity, some of which are conjugative, occur widely in lactic streptococci (21, 22, 23, 24, 25, 26, 27, 28). Although powerful R-M systems have been achieved by the additive effect of two plasmids (21, 24) the potential of R-M as a defence mechanism against phage is limited since the insensitivity conferred is not complete and phage which become modified (i.e. protected from subsequent restriction) pose a threat to the host that previously restricted the unmodified phage.

A plasmid pNP40 originating in Streptococcus lactis subsp. diacetylactis DRC3 has been described by McKay L.L. and Baldwin K.A. (1). The mechanism associated with this plasmid was effective at 21 and 32°C but not at 37°C. Furthermore, evidence was presented that pNP40 was thermosensitive in its replication. Klaenhammer et. al. have described a plasmid pTR2030, originating in S. lactis ME2, which encodes a heat-sensitive phage-defence mechanism which limits the burst size and plaque size of specific phage without altering the efficiency of plaquing (e.o.p.) or the level of adsorption (2). This plasmid is described in European Specification No. 208468A2. The pTR 2030 mechanism was heat sensitive in an S. lactis background but not in S. cremoris hosts when challenged with isometric-headed phage (2, 3). The effect of high temperature on the activity of the pTR2030 associated mechanism against prolate-headed phage was not assessed. pTR2030 could be conjugally transferred to a range of S. cremoris strains (3, 4) and it was established that the plasmid conferred resistance to small isometric-headed phage. However, prolate- and large isometric-headed phage were either not inhibited in the pTR2030 transconjugants or exhibited a reduction in plaque size without a reduction in e.o.p. (5). The presence of pTR2030 in a lysogen was shown to be unable to inhibit prophage induction in S. cremoris R1 but it did prevent the lysogenic phage from infecting a pTR2030-containing prophage cured derivative S. cremoris R1 (6). It was suggested that the phage resistance mechanism acts at the cell surface or membrane to prevent small isometric-headed phage DNA passage into the host cell or inhibits early events required for lytic replication of externally infecting phage (6). Sanders et al. have devised a conjugal strategy for the construction of fast acid-producing, bacteriophage-resistant lactic streptococci for use in dairy fermentations by introducing pTR2030 into lactose-utilising, antibiotic-sensitive recipient strains (7). This report also describes the use of deletion analysis to locate the region of pTR2030 encoding phage resistance and the cloning of a specific HindIII fragment in E. coli using the pBR322 vector plasmid. A 150 bp region of pTR2030 was sequenced and 19 base pairs from within this region were chemically synthesized to be used as a very specific probe to identify strains harbouring pTR2030.

References 4, 5, 6 and 7 and European Specification No. 208 468A2 were published after the priority date of the present application.

Although there has been considerable progress in the study of plasmid-encoded phage insensitivity mechanisms, there is still a definite need for more potent systems. In particular, a heat insensitive mechanism active against virulent prolate-headed phage in S. cremoris and S. lactis backgrounds is highly desirable for use in the food industry.

It is an object of the present invention to provide plasmids which encode phage-insensitivity in species useful in cheese manufacture and other areas of the food industry.

The invention provides an isolated plasmid carrying one or more genes which are capable of conferring insensitivity to one or more phages upon Streptococcus cremoris bacteria, the resistance to such phages not being affected at temperatures of 35°C to 40°C, characterised by genes which are also capable of conferring phage insensitivity upon Streptococcus lactis or Streptococcus lactis subsp. diacetylactis, the capability of conferring phage insensitivity not being destroyed by temperatures up to 39°C.

The plasmids of the present invention are isolated plasmids.

The term "isolated plasmid" as used herein means a plasmid which has been removed from the original host in which it was identified, and progeny and derivatives thereof, having the same phage insensitivity properties and includes such plasmids both when in a bacterium- free state and when they have been introduced into another bacterial cell.

In particular the invention provides a plasmid as defined above originating in a Streptococcus cremoris or a Streptococcus lactis strain, and a plasmid which confers insensitivity to both small isometric-headed and prolate-headed phage.

The invention further provides a recombinant plasmid carrying genes which encode lactose utilization and phage insensitivity in lactic streptococci, such a plasmid being generated by in vivo cloning.

The invention also provides a recombinant plasmid carrying genes which encode phage insensitivity, lactose utilization and high-frequency conjugative ability in lactic streptococci, such a plasmid being generated by in vivo cloning.

In particular aspects, the invention provides plasmids

pCl 750

pCl 777

pCl 829

pCl 528

isogenic derivatives thereof; plasmids which are substantially similar thereto, particularly plasmids having at least about 80% homology therewith; and DNA in such plasmids encoding phage insensitivity with or without lactose utilization and with or without high frequency conjugative ability.

The invention also provides bacterial hosts, particularly lactic streptococcal hosts into which has(ve) been introduced one or more of the plasmids defined above; more particularly a host into which have been introduced two of the plasmids defined above whose effect on the phage insensitvity pattern is additive (i.e. in the case of phage which are only partially inhibited by each plasmid on its own, the presence of the two plasmids together gives substantially total insensitivity). In particular, the invention provides lactic streptococci hosts into which have been introduced plasmids pCl 750 and pCl 829. The invention also provides isogenic derivatives of these plasmids including Lac-marked derivatives thereof, plasmids which are substantially similar thereto, or plasmids having phage insensitivity encoding DNA which is substantially similar to that of pCl 750, pCl 829 and pCl 528.

The invention especially relates to all of the above-defined materials in bioligically pure form or essentially pure form.

The invention further provides dairy starter cultures, particularly cheese starter cultures, containing any of the materials defined above. The invention also has potential for use in cultures used in meat and meat fermentations such as the manufacture of "German" sausage, vegetable fermentation cultures and probiotic cultures.

The invention also provides methods of conferring phage insensitivity on a bacterium comprising introducing into the said bacterium a plasmid as defined above, and on a food starter culture comprising bacteria in which a plasmid as defined above is introduced into the said bacteria.

This application more particularly describes three plasmids which confer valuable phage insensitivity against a variety of phage including virulent prolate-headed phage and offer advantages over other plasmids described in the literature. For example, some of the prior art plasmids are non-conjugative and cannot easily be used to construct phage insensitive starter strains. The plasmids pNP40 (1) and pTR2030 (2-7) are both heat-sensitive in a S. lactis background although the latter was heat-insensitive in S. cremoris hosts when titred against small isometric-headed phage. In contrast, the plasmids described in this application encode heat insensitive mechanisms effective against prolate-headed phage in both S. cremoris and S. lactis backgrounds. This is of major importance in commercial cheese-making.

4

EP 0 246 909 B1

pCI528, based on its ability to inhibit isometric- and prolate-headed phage, is the most potent phage insensitivity plasmid currently available. Furthermore, the additive effect of the mechanisms associated with pC1750 and pCI829 against prolate-headed phage has very significant practical applications.

From a mechanistic viewpoint, pCI750 differs from pTR2030 in that the latter does not inhibit the induction of prophage when present in a lysogenic host whereas pC1750 will cause a very severe reduction in the levels of inducibility. Furthermore, the presence of pTR2030 in a host is unable to reduce the e.o.p. of prolate-headed phage. pCI750 and pCI829 can reduce the e.o.p. by between 0.5 and seven log cycles. pCI528 is different to pNP40 and pTR2030 in that an effect on phage adsorption contributes to its overall phage insensitivity mechanism.

Brief Description of the Drawings

The present invention will now be described with reference to the accompanying drawings which show the following:-

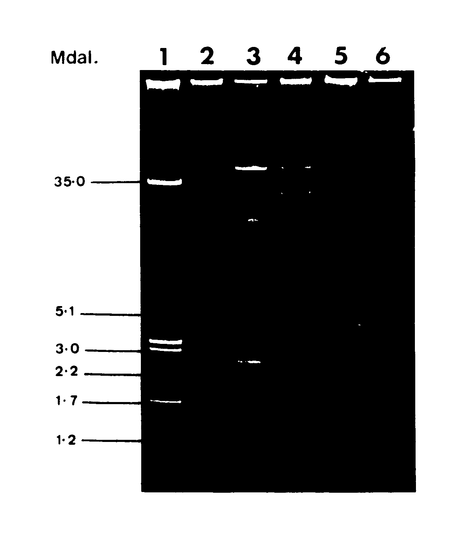

Fig. 1    Agarose gel (0.7%) showing plasmids from S. cremoris UC653 and selected Lac[+] phage insensitive transconjugants.
Lane 1 - E. coli V517 molecular weight standards
Lane 2 - Plasmid-free S. lactis MG1363 Sm recipient
Lane 3 - S. cremoris UC653 donor containing 6 plasmids of 44, 26, 15, 10, 2.6 and 1.6 Mdal.
Lane 4 - S. lactis AB001 containing 43.6 Mdal pCI750 and 26 Mdal pCI726
Lane 5 - S. lactis AB003 containing 83 Mdal plasmid
Lane 6 - S. lactis AB002 containing 77 Mdal plasmid

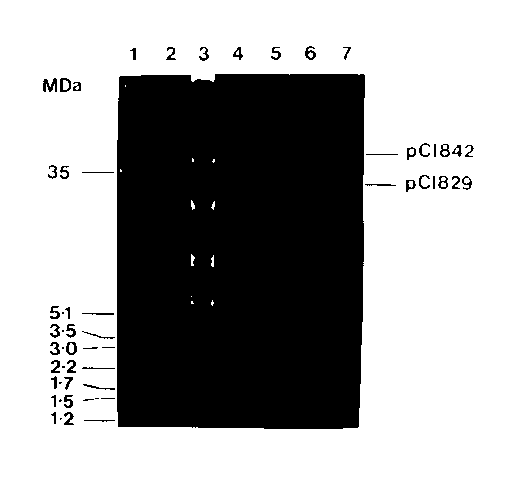

Fig. 2    Agarose gel (0.7%) showing plasmid profiles of S. lactis UC811 and selected Lac[+] phage insensitive transconjugants
Lane 1 - E. coli V517 molecular weight standards
Lane 2 - Plasmid-free S. lactis MG1363 Sm recipient
Lane 3 - S. lactis UC811 donor containing plasmids of 42, 27, 24.5, 15, 12.5, 6.5, 5.0, 3.6, 2.0, 1.5, 1.4 Mdal
Lane 4 - Transconjugants AC001 harbouring 27 Mdal pCI829 and 42 Mdal pCI842
Lanes 5-7 Selected transconjugants harbouring different plasmids from strain UC811

Fig. 3    Construction of an S. lactis strain harbouring two phage insensitive plasmids - pCI829 and pCI751.
Lane 1 - E. coli V517 molecular weight standards
Lane 2 - Lac[−] AC001 harbouring pCI829 alone and used as a recipient
Lane 3 - Donor strain AB012 harbouring pCI751
Lane 4 - S. lactis AC003 containing pCI751 and pCI829

Fig. 4    Restriction maps of the phage insensitive plasmids pCI750 and pCI829

Fig. 5    Plasmid shuttle vector pAM401 used for cloning fragments of pCI829. (from Wirth et al. - see ref. 18)

Fig. 6    Agarose gel showing EcoR1 digested pCI829 and recombinant plasmids following cloning in pAM401
Lanes 1, 3, 4 and 5 - EcoR1 digested recombinant plasmids showing linearised pAM401 (10.4 kb) and fragments from pCI829 of 2.5, 2.1, 1.7 and 0.7 kb
Lane 7 - EcoR1 digest of pCI829 showing fragments of 21, 12, 2.5, 2.1, 1.7, 0.8, 0.7 and 0.4 kb
Lane 8 - Undigested pCI829
Lane 9 - Lambda EcoR1/HindIII Standard digest

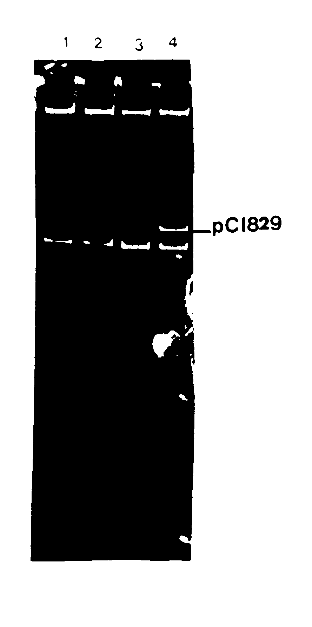

Fig. 7    Agarose gel (0.7%) showing plasmid profiles of S. cremoris UC503 and selected plasmid cured derivatives
Lanes 1-3 - Phage sensitive derivatives of S. cremoris UC503 cured of the 28 Mdal pCI528 plasmid.
Strain UC563 is shown in lane 2.
Lane 4 - S. cremoris UC503 harbouring plasmids of 46, 33, 28, 17, 4.0 and 2.1 Mdal

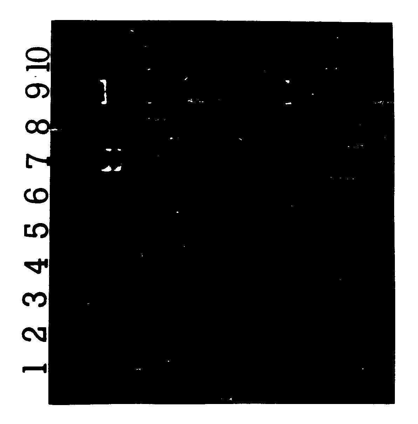

Fig. 8    Agarose gel (0.7%) showing the construction of a strain of S. lactis harbouring the phage insensitive plasmid pCI528 from S. cremoris UC503.
Lane 1 - S. cremoris UC503
Lane 2 - S. cremoris UC563 cured of pCI528
Lane 3 - S. cremoris UC503 containing pAMβ1
Lane 4 - S. lactis UC504 - i.e. S. lactis transformant containing pCI528, pAMβ1 and two cryptic

5

plasmids from strain UC503
Lane 5 - S. cremoris UC504 cured of pAMβ1
Lane 6 - S. lactis 505 cured of pLP712
Lane 7 - S. lactis 505 containing pLP712 and pCI829
Lane 8 - S. lactis MG1299 containing pLP712
Lane 9 - E. coli V517 molecular weight standards
Lane 10- S. lactis MG3020 containing pAMβ1

PLASMID:

A plasmid is an extrachromosomal DNA molecule capable of automomous replication.

Plasmids

pCI750 is a 43.6 Mdal.(66Kb) plasmid originally identified in S. cremoris UC653. This plasmid can be conjugatively transferred to Group N streptococcal recipients where it confers insensitivity to bacteriophage infection. S. lactis AB001 strain containing this plasmid has been deposited at the National Collection of Industrial Bacteria (NCIB), Aberdeen, Scotland under Accession No. 12261 on 16th May 1986.

pCI 777 is a 77Mdal plasmid generated following in vivo cloning of lactose utilization genes in the phage insensitivity plasmid after a conjugation experiment between S. cremoris UC653 and S. lactis MG1363Sm. It encodes phage insensitivity, lactose utilization and high frequency conjugative ability. S. lactis AB002 strain containing this plasmid has been deposited at the NCIB under Accession No. 12262 on 16th May 1986. It is representative of plasmids pCI 751 and pCI 783.

pCI 751 is a 51Mdal plasmid generated following in vivo cloning of lactose utilization genes in the phage insensitivity plasmid after a conjugation experiment between S. lactis AB001 and S. lactis LM 0230 Sp. It encodes phage-insensitivity, lactose utilization and high frequency conjugative ability.

pCI 783 is an 83Mdal plasmid generated following in vivo cloning of lactose utilization genes in the phage insensitivity plasmid after a conjugation experiment between S. cremoris UC653 and S. lactis MG1363Sm. It encodes phage insensitivity and lactose utilization.

pCI829 is a 27Mdal (44Kb) plasmid originally identified in Streptococcus lactis UC811 (formerly BA2). This plasmid can be conjugatively transferred to Group N streptococcal recipients where it confers insensitivity to bacteriophage infection. S. lactis AC001 strain containing this plasmid has been deposited at the NCIB under Accession No. 12263 on 16th May 1986.

pCI528 is a 28Mdal (43 Kb) plasmid originally identified in S. cremoris UC503. This plasmid can be introduced to Group N streptococcal recipients either by transformation or conjugation where it confers insensitivity to bacteriophage infection. S. lactis UC505 strain containing this plasmid has been deposited at the NCIB under Accession No. 12478 on 18th May 1987.

Group N Streptococci

This group of bacteria includes three species of the genus Streptococcus - S. lactis, S. cremoris and S. lactis, subsp. diacetylactis. These bacteria may also be termed the mesophilic lactic streptococci.

S. cremoris UC653

S. cremoris UC653 is a strain originally isolated from a mixed strain starter culture. This strain has been used as a component of a defined strain starter used in commercial Cheddar cheesemaking and is deposited in the Stock Culture Collection, Dairy and Food Microbioloby Department, University College, Cork. It has also been deposited at the NCIB under Accession No. 12265 on 20th May 1986.

S. cremoris UC503

S. cremoris UC503 is a strain originally isolated from a mixed strain starter culture used in commercial Cheddar cheesemaking. It was deposited at the NCIB under Accession No. 12477 on 18th May 1987. This strain was previously named S. cremoris F.

S. cremoris UC563

S. cremoris UC563 is a derivative of S. cremoris UC503 cured of its 28 Mdal phage insensitivity plasmid following acriflavin (25 μg/ml) treatment. This strain is significantly more sensitive to phage UC503 and is also sensitive to phage c2 and UC811 which are incapable of plaquing on S. cremoris UC503.

S. lactis UC811 (formerly BA2)

S. lactis UC811 is a classical "laboratory strain" and is deposited in the Stock Culture Collection, Dairy and Food Microbiology Department, University College, Cork. It has also been deposited at the NCIB under Accession No. 12266 on 20th May 1986. This strain was previously named S. lactis BA2.

S. lactis MG1363 Sm

S. lactis MG1363 Sm is a plasmid-free derivative of S. lactis 712 obtained from M. Gasson, Food Research Institute, Reading, U.K. and made resistant to Streptomycin in the present inventors' laboratory. This strain was used as a recipient in conjugation experiments.

S. lactis MG1229

S. lactis MG1229 is a derivative of S. lactis 712 cured of all its native plasmids except pLP712, a Lac/Prt plasmid. This strain was obtained from M. Gasson, Food Research Institute, Reading, U.K. and was used as a recipient in conjugation experiments.

S. lactis MG3020

S. lactis MG3020 is a derivative of S. lactis MG1363 Sm containing the broad host range plasmid pAMβ1 (17 Mdal). In addition to the erythromycin (Em) resistance encoded on pAMβ1, this strain is resistant to streptomycin and was obtained from M. Gasson, Food Research Institute, Reading, U.K. This strain was used as a donor of pAMβ1 in conjugation experiments.

S. lactis LM0230 Sp

S. lactis LM0230 Sp is a plasmid-free bacteriophage-sensitive derivative of S. lactis C2 obtained from L.L. McKay, University of Minnesota, St. Paul, Minnesota, USA. This strain was used as a recipient in conjugation experiments.

S. lactis LM2305

S. lactis LM2305 is a plasmid-free, bacteriophage-sensitive neomycin sulphate resistant isolate of S. lactis C2 obtained from L.L. McKay, University of Minnesota, St. Paul, Minnesota, USA. This strain was used as a recipient in conjugation experiments.

S. lactis MMS366

S. lactis MMS366 is a Lac⁻ bacteriophage sensitive recombination deficient (Rec⁻) rifampin resistant isolate of S. lactis ML3 containing plasmids of 31.9, 5.6 1.98 and 1.0 Mdal, obtained of L.L. McKay, University of Minnesota, St. Paul, Minnesota, USA. This strain was used as a recipient in conjugation experiments.

S. lactis LM 3301

S. lactis LM 3301 is a Lac⁻, bacteriophage sensitive, Streptomycin resistant derivative of S. lactis ML3 containing plasmids of 31.9, 5.6, 1.98 and 1.0, obtained from L.L. McKay, University of Minnesota, St. Paul, Minnesota, USA.

S. lactis SK3 Lac⁻ Tc^R

S. lactis SK3 Lac ⁻Tc^R is a lactose negative (Lac⁻) tetracycline resistant (Tc^R) derivative of S. lactis SK3. The strain was made Lac⁻ following subculture at 37°C and made Tc^R after the conjugal introduction of the transposon Tn919 in the present inventors' laboratory. S. lactis SK3 Lac⁻Tc^R was used as a recipient in conjugation experiments.

S. lactis subsp. diacetylactis 18-16 Lac⁻Tc^R

S. lactis subsp. diacetylactis 18-16 Lac⁻Tc^R is a Lac⁻Tc^R derivative of S. lactis subsp. diacetylactis 18-16 constructed in a manner identical to that described above for S. lactis SK3 Lac⁻Tc^R. S. lactis subsp. diacetylactis 18-16 Lac⁻Tc^R was used as a recipient in conjugation experiments.

S. lactis subsp. diacetylactis 18-16 Lac⁻S

S. lactis subsp. diacetylactis 18-16 Lac⁻S is a Lac⁻ streptomycin resistant derivative of S. lactis subsp. diacetylactis 18-16 and was used as a recipient in conjugation experiments.

S. cremoris UC411 Lac⁻Fus^R

S. cremoris UC411 Lac⁻Fus^R is a Lac⁻ (made by subculture at 37°C) fusidic acid resistant (Fus^R) derivative of S. cremoris UC411. This strain was made resistant to Fus by plating $10^9$ cells on plates containing 25 ug/ml Fus and selecting spontaneous resistant mutants.

Transformant

Transformants are defined as cells arising from the introduction of naked DNA (plasmid DNA in the case of this study) into protoplasted recipients.

S. lactis UC504

S. lactis UC504 is a transformant arising from the treatment of protoplasts of S. lactis MG1363 Sm with total plasmid DNA from S. cremoris UC503 (pAMβ1). S. lactis UC504 harbours pCI528 (28 Mdal), pAMβ1 (17 Mdal) and two cryptic plasmids of 18.0 and 2.1 Mdal from S. cremoris UC503.

Transconjugant

Transconjugants are defined as cells arising from the conjugative transfer of DNA from a donor to a recipient bacterium.

S. lactis AB001

This is a transconjugant arising from a conjugation experiment between the S. cremoris UC653 donor and the S. lactis MG1363Sm recipient. S. lactis AB001 harbours plasmids of 26 Mdal (pCI726, encodes ability to utilize lactose) and 43.6 Mdal (pCI750, encodes bacteriophage insensitivity). It has been deposited at the NCIB under Accession No. 12261 on 16th May 1986.

S. lactis AB002

This is a transconjugant arising from a conjugation experiment between the S. cremoris UC563 donor and the S. lactis MG1363 Sm recipient. S. lactis AB002 harbours a single plasmid of 77 Mdal (pCI777, encodes both lactose utilization and bacteriophage insensitivity). This strain exhibits a high frequency conjugative capacity. It has been deposited at the NCIB under Accession No. 12262 on 16th May 1986.

S. lactis AB003

This is a transconjugant arising from a conjugation experiment between the S. cremoris UC653 donor and the S. lactis MG1363 Sm recipient. S. lactis AB003 harbours a single plasmid of 83 Mdal (pCI783, encodes both lactose utilization and bacteriophage insensitivity).

## S. lactis AC001

This is a transconjugant arising from a conjugation experiment between the S. lactis UC811 donor and the S. lactis MG 1363 Sm recipient, harbouring plasmids of 27 Mdal (pCI829, encodes bacteriophage insensitivity) and 42 Mdal (pCI842, encodes lactose utilization). It has been deposited at the NCIB under Accession No. 12263 on 16th May 1986.

## S. lactis AC003

This is a transconjugant arising from a conjugation experiment between the S. lactis AB012 donor and the S. lactis AC001 Lac⁻ recipient. S. lactis AC003 harbours plasmids of 51 Mdal (pCI 751, encodes both lactose utilization and bacteriophage insensitivity) and 27 Mdal (pCI 829, encodes bacteriophage insensitivity).

## S. lactis MM20

S. lactis MM20 is a transconjugant arising from a conjugation experiment between the S. cremoris UC653 donor and S. lactis MMS366 recipient harbouring plasmids of 26 Mdal (pCI726, encodes lactose utilization genes) and 43.6 Mdal (pCI750, encodes bacteriophage insensitivity).

## S. lactis MM21

S. lactis MM21 is a transconjugant arising from a conjugation experiment between the S. cremoris UC653 donor and the S. lactis MMS366 recipient harbouring a single plasmid of 60 Mdal (pCI760, encodes both lactose utilization and bacteriophage insensitivty).

## S. lactis UC505

S. lactis UC505 is a transconjugant arising from a conjugation experiment between S. lactis UC504 donor and the S. lactis MG1299 recipient. This transconjugant harbours pCI 528 (28 Mdal) and pLP 712 (33 Mdal). It has been deposited at the NCIB under Accession No. 12478 on 18th May 1987.

## E. coli LE392 (pAM401)

This strain was used as source of the pAM401 cloning vector. This plasmid, originally obtained from D. Clewell, University of Michigan, Ann Arbor, M1, USA, was introduced into E. coli LE392 in the present inventors' laboratory.

## Bacteriophage (phage)

Bacteriophage are viruses capable of infecting bacteria.
Phage 712, SK1, StL5, Eb1 and c2 are capable of infecting (i.e. lytic for) S. lactis MG1363 Sm. The phage c2 is a prolate-headed phage (29), while the phage 712 is generally accepted in the field to be a small isometric-headed phage and was shown to be so by morphological examination in the present inventors' laboratory.
Phages 18 - 16 and drc3 are capable of infecting S. lactis subsp. diacetylactis 18 - 16S.
Phages uc503 infects S. cremoris UC503 while phages uc503, c2 and UC811 infect mutants cured of the 28 Mdal plasmid. Phages sk3 and uc411 are capable of infecting hosts S. lactis SK3 Lac⁻Tc$^R$ and S. cremoris UC411 Lac⁻Fus$^R$, respectively.

## Bacterial strains, bacteriphage and media

The bacterial strains were routinely grown in the M17 medium of Terzaghi and Sandine (8) in which glucose replaced lactose when required (GM17). Solid media contained 1.5% agar (Oxoid No. 3). The antibiotics (Sigma, U.K.) streptomycin (Sm) and spectinomycin (Sp) were added to selective media at levels of 600 $\mu$g/ml and 150 $\mu$g/ml, respectively. The method used to generate spontaneous antibiotic insensitive mutants of lactic streptococci has been previously reported by Hill et al. (9).

## Conjugation experiments

Agar surface matings were carried out as described by McKay et al. (10). Lactose indicator agar (LIA) of McKay et al. (11) containing the appropriate antibiotic was used to select for Lac transfer. Sensitivity of Lac$^+$ transconjugants to phage was determined using the spot test method. Briefly, this involved incorporating 0.1 ml of the fully grown test cultures into an M17 soft agar (0.7%) overlay which was poured on a hard M17 agar (1.5%) plate. After solidifying, 10 $\mu$l aliquots of the appropriate phage suspension ( $10^8$ pfu/ml) were spotted onto the overlay. Results were read after overnight incubation at 30°C.

## Frequency of transfer

The frequency of transfer of a specific marker (i.e. Lactose utilization or phage insensitivity) refers to the number of transconjugants obtained divided by the number of recipients and is described as the frequency per recipient.

## Transformation

Protoplasts of S. lactis MG1363 Sm were made, transformed and regenerated as described by Gasson and Anderson (12). The transforming DNA was total plasmid DNA from S. cremoris UC503 (pAM$\beta$1) and transformants were selected on the basis of Em resistance.

## Assay of phage sensitivity.

Sensitivity of specific strains to bacteriophage was determined using a "spot test" whereby 0.1 ml of a fully grown culture was incorporated into an M17 soft agar (0.7%) overlay, which was poured on a hard M17 agar (1.5%) plate. After solidifying, 10 ul of a phage suspension ($10^8$ pfu/ml) was spotted on the overlay. Results were read after overnight incubation at 30°C.

## Determination of effect of temperature on phage insensitivity mechanism.

The effect of elevated temperature (up to 40°C) on the phage insensitivity mechanisms was determined by assaying phage (preheated to the appropriate temperature) on the culture (preheated) and then incubating the assays at the desired temperature.

## Milk activity tests.

The activity test described by Heap and Lawrence (4) was used to determine the effectiveness of the phage insensitivity mechanisms in simulated laboratory cheesemaking trials.

## Bacteriophage adsorption

0.6 ml of the phage suspension ($10^5$ pfu/ml) was added to a 1.5 ml microcentrifuge tube containing 0.2 ml CaCl$_2$ (0.185M) and 0.6 ml of the fully grown test culture resuspended in M17 (pH 7.5). The tube was mixed vigorously for 10 sec and then incubated at 30°C for 12 min to allow adsorption to occur. After cooling on ice to stop the reaction, the tubes were centrifuged in a microfuge (Eppendorf) for 10 min to pellet the cells and adsorbed phage. A 1.0 ml sample of the supernatant was removed and titred for phage using a sensitive host as indicator. Percent phage adsorbed was determined as [(control titre - residual titre)/control titre] x 100.

## Isolation and analysis of plasmid DNA

The method of Anderson and McKay (14) was used to isolate plasmid DNA from streptococci and E. coli V517. Plasmid profiles were analysed by electrophoresis on 0.7% agarose (Sigma, U.K.) gels using a vertical gel apparatus. Electrophoresis was carried out in TAE buffer (40 mM Tris, 10 mM Na$_2$EDTA, 12mM sodium acetate, pH 7.8). Gels were strained in ethidium bromide solution (5 $\mu$g/ml) and DNA was visualised with short-wave UV light.

Restriction mapping, hybridization analysis and molecular cloning

Digestions with restriction endonucleases were carried out as described by Maniatis et al. (15). Restriction maps of pCI 750 and pCI 829 were prepared as described by Coveney et al (16).

The Southern blot and hybridization techniques have been described by Southern (17) and Coveney et al. (16). EcoRI fragments of pCI 829 were shotgun cloned in E. coli using the vector pAM401 and employing the cloning methodologies described by Maniatis et al. (15). Clones containing recombinant plasmids were identified by random screeing of the plasmid contents of transformants.

**Results**

Conjugative transfer of lactose utilization and phage insensitivity from S. cremoris UC653 and S. lactis UC811

Markers associated with lactose utilization and phage insensitivity were transferred from S. cremoris UC653 and S. lactis UC811 donors to a S. lactis MG1363 Sm recipient in agar surface matings (10). The transfer frequencies are shown in Table 1.

Transconjugants from these matings were designated AB001, AB002 and AB003 or AC001 depending on whether they are derived from the strain UC653 or UC811 donors, respectively. All these transconjugants were totally resistant to phage 712 and allowed phage c2 to plaque with a reduced size. Both phage are lytic for strain MG1363.

Plasmid profile analysis of transconjugants derived from S. cremoris UC653 and S. lactis UC811 donors

The three Lac$^+$ phage 712 insensitive transconjugants isolated from the UC653 x MG1363 mating were lysed by the method of Anderson and McKay (14) and their plasmid profiles were analysed. S. lactis AB001 contained two plasmids of 43.6 Mdal (pCI750) and 26 Mdal (pCI726) while S. lactis AB002 and S. lactis AB003 each harboured single plasmids of 77 Mdal (pCI777) and 83 Mdal (pCI783), respectively (Fig. 1). The S. lactis AC001 transconjugant (also Lac$^+$, phage 712 insensitive) contained two plasmids of 27 Mdal (pCI829) and 42 Mdal (pCI842) (Fig. 2).

Further mating experiments between the AB001 and AC001 donors and a S. lactis LM0230 Sp recipient were completed (Table 1). Transconjugants showed the same resistance patterns as did AB001 and AC001 - i.e. partial insensitivity to phage c2 and total insensitivity to phage 712. A phenotypically representative transconjugant derived from the AB001 x LM0230 mating was designated S. lactis AB012 and this harboured a recombinant Lac/phage insensitivity plasmid of 51 Mdal (pC1751).

Analysis of the conjugative abilities of lactose utilization and phage insenstivity plasmids

Further mating experiments comparing the ability of AB001 and AB002 to act as donors showed that AB002 could transfer Lac (and phage insensitivity) at frequencies approximately four log cycles higher than AB001 (Table 2). This indicated that pCI777 which is present in S. lactis AB002 harbours genes conferring an ability to transfer at high frequency.

The pCI750 plasmid present in S. lactis AB001 was removed using plasmid curing treatments. Isolates missing this molecule reverted to phage 712 sensitivity. However, these isolates still harboured pCI726 and remained Lac$^+$ confirming that pCI726 encoded lactose utilization genes while pCI750 encoded phage 712 insensitivity. Furthermore, repeated attempts to transfer pCI726 to S. lactis LM2306 in conjugation experiments were unsuccessful indicating that pCI750 harboured genes conferring conjugative ability in addition to phage insenstivity.

A conjugation experiment between the S. cremoris UC653 donor and the S. lactis MMS336 recipient (which is recombination-deficient) also yielded Lac$^+$, phage 712 insensitive transconjugants (Table 2). However, these were of two types, (a) S. lactis MM20 contained two plasmids of 43.6 Mdal (i.e. pCI750) and 26 Mdal (i.e. pCI726) and (b) S. lactis MM21 contained a single plasmid of 60 Mdal. This latter result shows that recombination between pCI750 and pCI726 (or parts thereof) can still occur in a Rec$^-$ strain suggesting that recombination is Rec independent.

Introduction of the phage insensitivity mechanism encoded on pC1750 to a strain harbouring pCI829 i.e. construction of a strain containing two phage insensitivity plasmids

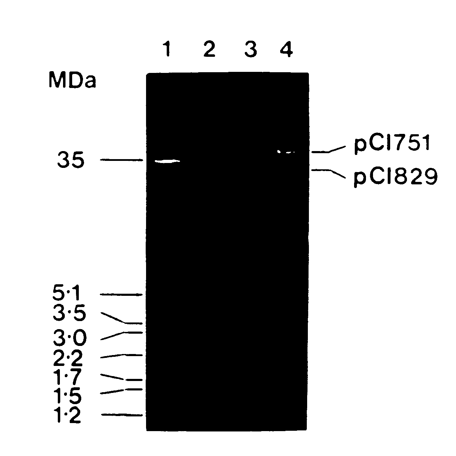

The phage insensitivity genes encoded on pCI750 were introduced on pCI751 (derived from pCI750 and containing lac genes) into a Lac⁻ derivative of S. lactis AC001 (which still contains pCI829) in conjugation experiments generating transconjugant AC003 (Fig. 3). The effect of the presence of both mechanisms in a single strain was to confer total insensitivityto phage c2 (which was capable of forming plaques on strains harbouring either pCI750 or pCI829 alone) in addition to continuing insensitivty to phage 712. This effect is shown in Table 9 where isolates harbouring pCI750 or pCI829 alone allowed phage c2 to form plaques of 1.5 mm diameter (which are significantly smaller than those formed on strain MG1363 Sm which is plasmid-free). However, all plaquing ability is eliminated in the case of transconjugant AC003 which contains the phage insensitivity mechanisms encoded on both plasmids.

Extent and effectiveness of phage insensitivty conferred by pCI750 and pCI829

The effect of the phage insensitivity plasmids pCI750 and pCI829 on an extended range of phage was examined (Table 3). Plasmid pCI750, present in S. lactis AB001 was capable of conferring total insensitvity to phage sk1 in a manner identical to the insensitivity conferred against phage 712. Also, the plaque sizes developed by all phage that were capable of plaquing on any of the hosts containing pCI750, pCI829 or the mechanisms encoded by both pCI750 and pCI829 together, were very significantly reduced in size compared to those formed on S. lactis MG1363 Sm (which contains no plasmids). In all cases the reduction in plaque size was greater than 50%. Furthermore, the titre of all the phage virulent for hosts AB001, AC001 and AC003 (with the sole exception of phage m13 plaqued on AC001) were all reduced by between 0.5 and 7 log cycles compared with those on S. lactis MG1363 Sm, suggesting that a second mechanism of insensitivity was encoded on these plasmids (i.e. in addition to the mechanism resulting in elimination or reduction of plaque size, Table 3). The manufacturing process in Cheddar cheese-making involves a cooking step where the temperature is increased from 30°C to 37°C or greater. The data in Table 3 indicates that temperatures of 37°C or 40°C have no adverse effect on the ability of either pCI750 or pCI829 to inhibit phage replication. This means that both plasmids will be suitable for inclusion in strains used in commercial Cheddar cheese-making.

Morphological examination of some of the phage described in Table 3 showed that 712 is a member of the commonly occurring isometric-headed phage group. However, phages c2 and m13 both have prolate heads (29) and thus are members of the extremely virulent prolate-headed grouping. This may explain why these phage can overcome the protective effect of a single phage insensitivity plasmid. Nevertheless, it is significant that the presence of two plasmids can confer the high degree of insenstivity observed in this study.

Transfer of the phage insensitivity plasmids to selected strains of S. lactis, S. cremoris and S. lactis subsp. diacetylactis

The transconjugants AB002 (containing the Lac/phage insensitivity recombinant plasmid pCI777) and AC003 (containing the phage insensitivity plasmid pCI829 and the recomibinant Lac/phage insensitivity plasmid pCI751) were used as donors in a series of conjugation experiments with selected recipients (Table 4). In all cases the recipients were Lac⁻ (isolated following curing treatments at 37°C) and contained either the tetracycline resistance transposon Tn919 (in the case of strains SK3 and 18-16) or were spontaneous fusidic acid (Fus) resistant mutants (in the case of strain UC411) to facilitate selection of transconjugants. In all cases, transfer of the Lac marker was detected at a reasonably high frequency and a high proportion of these Lac⁺ transconjugants were also insensitive to the homolgous phage of the original recipient strain (Table 4). These data demonstrate that the phage insensitivity trait can be transferred by conjugation to a range of recipient types.

Milk activity tests

The lactose utilization genes of pCI726 and the phage insensitivity mechanism of pCI750 were introduced into S. lactis subsp. diacetylactis 18-16 S in conjugation experiments from the S. cremoris UC653 donor (frequency of Lac transfer = $1.6 \times 10^{-6}$ per recipient of which 23% were totally insensitive to phages 18-16 and drc3 which are highly lytic for the 18-16 wild-type strain). Selected phage insensitive 18-16 transconjugants were subjected to milk activity tests to determine the efficiency of the phage insensitivity mechanism in this strain when the culture was subjected to repeated growth cycles in milk at 30 and 37°C in the presence of both phage. Under these simulated cheese-making conditions, the presence of phage 18-16 and drc3 had no effect on the ability of the phage insensitive 18-16 transconjugants to produce normal levels of acid. This result indicated that the phage insensitivity mechanism of pCI750 will have a practical application in commercial practice.

Nature of the phage insensitivity mechanism associated with pCI750 and pCI829

Adsorption tests have shown that the presence of either pCI750 or pCI829 in an S. lactis MG1363 Sm host has no effect on the ability of the phage to adsorb. Furthermore, preliminary studies have been undertaken in the case of pCI750 to demonstrate its effect on the inducibility of prophage. The introduction of pCI750 into a lysogenic host reduced the inducibility of prophage compared to a control not containing the phage-insensitivity plasmid, suggesting that the associated mechanism interferes with DNA replication, protein synthesis or even a later stage in the infectious cycle. This contrasts with the reported effect of pTR2030 from Klaenhammer's laboratory, where it did not interfere with the inducibility of prophage and an effect at the level of the cell surface, cell membrane or some early stage in the lytic cycle was proposed as the mode of action of the plasmid (6).

Restriction mapping of the phage insensitivity plasmids pCI750 and pCI829

An analysis of plasmids pCI750 and pCI829 was carried out using a variety of restriction enzymes. The restriction data is as follows:-

### RESTRICTION ANALYSIS OF pCl750

| Restriction Enzyme | Number of Fragments | Fragment Size (kb) |
|---|---|---|
| BamHI | 2 | 35.6, 30.4 |
| BclI | 7 | 21.32, 13.77, 12.55, 9.75, 4.78, 2.13, 1.69 |
| BglII | Many | Not determined |
| BstEII | 2 | 56.74, 9.26 |
| EcoRV | 6 | 41.69, 11.1, 5.48, 5.26, 1.45, 1.08 |

| | | |
|---|---|---|
| HindIII | 11 | 23.22, 12,18, 6.70, 5.08, 4.13, 3.51, 3.28, 2.43, 2.03, 1.85, 1.60 |
| HpaII | 10 | 20,97, 14.51, 10.26, 5.91, 3.35, 3.10, 2.25, 2.19, 1.70, 1.55 |
| PvuII | 5 | 26.01, 20.44, 9.66, 6.54, 8.35 |
| SalI | 4 | 25.33, 20.06, 13.16, 7.51 |

| RESTRICTION ANALYSIS OF pC1829 | | |
|---|---|---|
| Restriction Enzyme | Number of Fragments | Fragment Size (kb) |
| SacI | 1 | 40.0 |
| StuI | 2 | 32.9, 6.9 |
| BglII | 3 | 20.8, 14.4, 4.0 |
| PvuII | 3 | 22.9, 8.3, 5.1, |
| ClaI | 4 | 21.2, 7.4, 6.1, 4.8 |
| EcoRV | 6 | 25.1, 5.9, 4.5, 3.1, 2.7, 2.1 |
| HaeIII | 4 | 25.1, 5.49, 5.0, 4.36 |
| HincII | 6 | 11.8, 5.6, 4.5, 3.8, 2.5, 1.7 |
| AvaI | 5 | 16.3, 12.5, 9.1, 4.6, 3.2 |
| HpaII | 8 | 12.6, 11.9, 10.8, 3.9, 2.4, 2.2, 1.2, 0.4 |
| EcoRI | 8 | 21.0, 12.1, 2.5, 2.1, 1.7, 0.84 0.69, 0.41 |

14

It will be noted that there may be small fragments of DNA which were undetected on the gels.

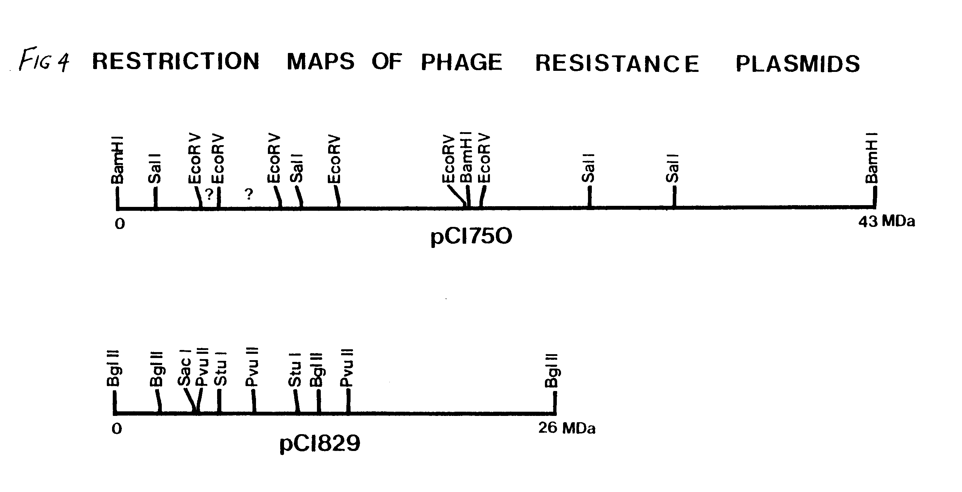

Preliminary restriction maps of pCI750 and pCI829 were constructed (Fig. 4). The pattern and sequence of the cleavage sites on the maps is typical of the particular plasmid and can be used very easily to identify the different molecules. A Southern blot and hybridization experiment, using pCI750 as a biotin-labelled probe showed no detectable homology between it and pCI829 suggesting that the two plasmids do not share any significant common sequences.

Cloning of fragments of pCI829 in E. coli

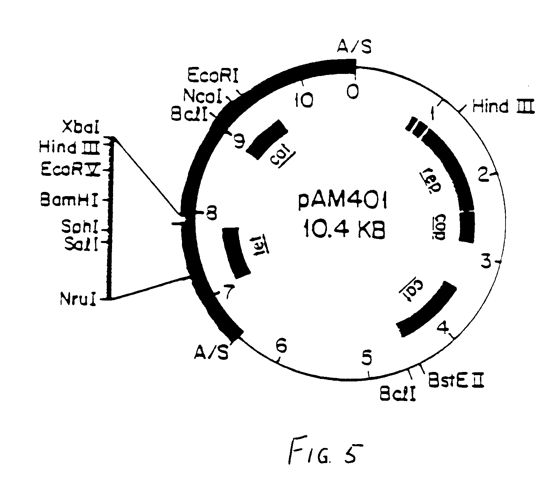

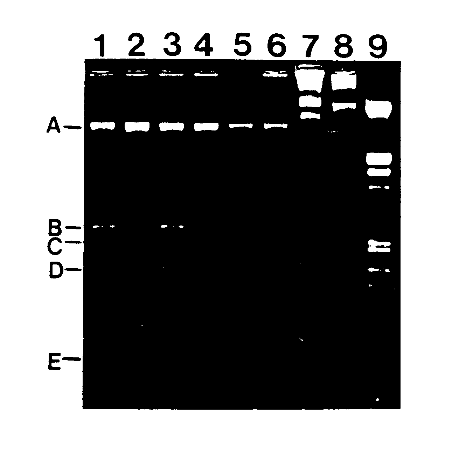

The restriction endonuclease EcoR1 digests the plasmid pCI829 into 8 fragments of 21.0, 12.1, 2.5, 2.1, 1.7, 0.84, 0.69 and 0.41 Kb. Using molecular cloning techniques, EcoR1 digested pCI829 DNA was shotgun cloned into the EcoR1 digested shuttle plasmid vector, pAM401 (Fig. 5, ref. 18). Following ligation in the presence of T4 ligase, the DNA was transformed into E. coli DB11. Transformants were selected on the basis of resistance to tetracycline, encoded on pAM401. Tetracycline resistant transformants arose at a frequency of $3 \times 10^2/\mu$g DNA and the plasmid profiles of random transformants were examined. EcoR1 digestion of recombinant plasmids showed that the 2.5, 2.1, 1.7, 0.84 and 0.69 kb EcoR1 fragments from pCI829 had been cloned (Fig. 6). Selected cloned fragments can be used to identify strains harbouring pCI829 (following the appropriate control experiments to ensure that the fragments do not hybridize to any resident plasmids in those strains).

Indentification of a bacteriophage insensitive plasmid from S. cremoris UC503

Experience in this laboratory has shown that S. cremoris UC503 displayed a high natural level of phage insensitivity. Mutants showing increased insensitivity to the homologous phage uc503 (i.e. a significant increase in plaque size) were readily isolated either spontaneously or after acriflavin (20 $\mu$g/ml) treatment. Analysis of the plasmid profiles of selected representative mutants showed the consistent loss of a 28 Mdal plasmid, pCI528 (Fig. 7). Furthermore, these mutants were also sensitive to c2 and uc811, phage which were incapable of plaquing on S. lactis UC503 which contains pCI528 (Table 5).

Introduction of the phage insensitivity plasmid pCI528 into S. lactis MG1363

It was not possible to transfer pCI528 from its S. cremoris UC503 host to an S. lactis MG1363 Sm recipient using the conjugative mobilization described earlier for plasmids pCI750 and pCI829. The introduction of the promiscuous plasmid pAM$\beta$1 as a mobilizing agent (from an S. lactis MG3020 donor) also failed to bring about transfer of pCI528. The alternative approach of using protoplast transformation was applied. Total plasmid DNA from S. cremoris UC503 containing pAM$\beta$1 was isolated and used to transform S. lactis MG1363 Sm protoplasts. Transformants were selected on the basis of resistance to erythromycin (Em) encoded by pAM$\beta$1 and the plasmid profiles of 50 individual isolates were examined. A single isolate, designated UC504, harboured a 28 Mdal plasmid (in addition to pAM$\beta$1 and two cryptic plasmids from strain UC503) (Fig. 8, lane 4). pAM$\beta$1 was eliminated from UC504 following heat curing treatments. pCI528 was removed from the presence of the two cryptic plasmids by conjugating it into S. lactis MG1299 (i.e. S. lactis MG1363 containing pLP712, a Lac/Prt plasmid from S. lactis 712). The plasmid profile of a transconjugant, designated UC505, containing both pCI528 and pLP712 is shown in Fig. 8 (lane 7).

Comparison of the phage sensitivity patterns of S. lactis strains UC505 and MG1363 Sm

S. lactis MG1363 Sm is sensitive to phage 712, c2, m13 and jj50 (Table 6). However, when these phage were titred against UC505 no plaques were formed by any of the phage, even though phage such as m13 had previously been shown to be extremely virulent. Increasing the temperature to 37° or 39°C had minimal or no effect on the insensitivity mechanism.

Examination of the adsorption of phage 712, c2, m13 and jj50 to UC505.

Adsorption tests showed that phages 712, c2, m13 and jj50 were capable of adsorbing to S. lactis MG1363 Sm at high efficiency (Table 7). However, the efficiencies of adsorption of the same phage to UC505 were between 35 and 45%. These data suggest that the phage insensitivity mechanism associated with pCI528 is different to those encoded by pCI750 and pCI829 and also to pTR2030 of Klaenhammer's group.

EP 0 246 909 B1

Milk activity tests comparing the ability of S. lactis strains MG1299 and UC505 to produce acid in the presence of homolgous phage.

Heap-Lawrence milk activity tests were performed to compare the ability of UC505 and MG1299 to produce acid in the presence of virulent phage. The data in Table 8 show that UC505 was capable of surviving 12 cycles of the test in the presence of high titres of phage 712, c2, m13 or jj50, or a cocktail of all four phage with no effect at all on acid producing ability. In contrast, MG1299 was unable to produce acid when exposed to any of the phage combinations.

**LITERATURE CITED**

1. McKay, L.L., and K.A. Baldwin. 1984. Conjugative 40 Mdal plasmid in Streptococcus lactis subsp. diacetylactis DRC3 is associated with resistance to nisin and bacteriophage. Appl. Environ. Microbiol. 47:68-74.

2. Klaenhammer, T.R. and R.B Sanozky. 1985. Conjugal transfer from Streptococcus lactis ME2 of plasmids encoding phage resistance, nisin resistance and lactose fermenting ability : evidence for a high-frequency conjugative plasmid responsible for abortive infection of virulent bacteriphage. J. Gen. Microbiol. 131:1531-1541.

3. Steenson, L.R. and T.R. Klaenhammer. 1985. Streptococcus cremoris M12R transconjugants carrying the conjugal plasmid pTR2030 are insensitive to attack by lytic bacteriphages. Appl. Environ, Microbiol. 50:851-858.

4. Sing, W.D. and T.R. Klaenhammer. 1986. Conjugal transfer of bacteriphage resistance determinants on pTR2030 into Streptococcus cremoris strains. Appl. Environ. Microbiol. 51:1264-1271.

5. Jarvis, A.W. and T.R. Klaenhammer. 1986. Bacteriophage resistance conferred on lactic streptococci by the conjugative plasmid pTR2030 : effects on small isometric-, large isometric-, and prolate-headed phages. Appl. Environ. Microbiol. 51:1272-1277.

6. Jarvis, A.W. and T.R. Klaenhammer. 1987. Bacteriophage resistance plasmid pTR2030 inhibits infection or rlt temperate bacteriophage but not induction of rlt prophage in Streptococcus cremoris Appl. Environ. Microbiol. 53:385-389.

7. Sanders, M.E., P.J. Leonhard, W.D. Sing and T.R. Klaenhammer. 1986. Conjugal strategy for construction of fast acid producing, bacteriophage-resistant lactic streptococci for use in dairy fermentations. Appl. Environ. Microbiol. 52:1001-1007.

8. Terzaghi, B.E. and W.E. Sandine. 1975. Improved medium for lactic streptococci and their bacteriophage. Appl. Environ. Microbiol. 29:807-813.

9. Hill, C., C. Daly and G.F. Fitzgerald. 1985 Conjugative transfer of the transpoon Tn919 to lactic acid bacteria. FEMS Microbiol. Lett. 30:115-119.

10. McKay, L.L., K.A. Baldwin and P.M. Walsh. 1980. Conjugal transfer of genetic information in group N streptococci. Appl. Environ. Microbiol. 40:84-91.

11. McKay, L.L., K.A. Baldwin and E.A. Zottola. 1972. Loss of lactose metabolism in S. lactis C2. Appl. Microbiol. 23:1090-1096.

12. Gasson, M.J. and P.H. Anderson. 1985. High copy number plasmid vectors for use in lactic streptococci. FEMS Microbiol. Lett. 30:193-196.

13. Heap, H.A. and R.C. Lawrence. 1976. The selection of starter strains for cheesemaking. N.Z.J. Diary Sci. Technol. 11:16-22.

14. Anderson, D.G. and L.L. McKay. 1983. Simple and rapid method for isolating large plasmid DNA from lactic streptococci. Appl. Environ. Microbiol. 46:549-552.

15. Maniatis, T., E.F. Fritsch and J. Sambrook. 1982. Molecular cloning: a laboratory manual. Cold Spring Harbor Laboratory, Cold Spring Harbor, N.Y.

16. Coveney, J., G.F. Fitzgerald and C. Daly. 1987. Detailed characterization and comparison of four lactic streptococcal bacteriophage based on morphology, restriction mapping, DNA homology and structural protein analysis. Appl. Environ. Microbiol. 53: In press (July, 1987).

17. Southern, E.M. 1975. Detection of specific sequences among DNA fragments separated by gel electrophoresis. J. Mol. Biol. 98:503-5517.

18. Wirth, R., F.Y. An and D.B. Clewell. 1986. Highly efficient protoplast transformation system for Streptococcus faecalis and a new Escherichia coli- S. faecalis shuttle vector. J. Bacteriol. 165:831-836.

19. Sanders, M.E. and T.R. Klaenhammer. 1983. Characterization of phage sensitive mutants from a phage insensitive strain of Streptococcus lactis : evidence for a plasmid determinant that prevents phage adsorption. Appl. Environ. Microbiol. 46:1125-1133.

20. de Vos, W.M., H.M. Underwood and F.L. Davies. 1984. Plasmid encoded bacteriophage resistance in Streptococcus cremoris SK11. FEMS Microbiol Lett. 23:175-178.

21. Chopin, A., M.C. Chopin, A. Moillo-Batt and P. Langella. 1984. Two plasmid determined restriction-modification systems in Streptococcus lactis. Plasmid 11:260-263.

22. Sanders, M.E. and T.R. Klaenhammer. 1981. Evidence for plasmid linkage of restriction and modification in Streptococcus cremoris KH. Appl. Environ. Microbiol. 42:944-950.

23. Wetzel, A., H. Neve, A. Geis and M. Teuber. 1986. Transfer of plasmid mediated phage resistance in lactic acid streptococci. Chem. Mikrobiol. Technol. Lebensm. 10:86-89.

24. Klaenhammer, T.R. and R. B. Sanozky-Dawes. 1986. A conjugal plasmid which confers phage restriction and modification activities to lactic streptococci. Abstract 222 from Second ASM Conference on Streptococcal Genetics, 21-24 May, Miami Beach, Florida, U.S.A.

25. Daly, C. and G.F. Fitzgerald. 1987. Mechanisms of bacteriophage insensitivity in the lactic streptococci. In Streptococcal Genetics (Ferretti, J.J. and R.C. Curtiss III, eds.) American Society for Microbiology, Washington D.C. U.S.A.

26. Klaenhammer, T.R.. 1984. Interactions of bacteriophages with lactic streptococci. Adv. Appl. Microbiol. 30:1-29.

27. Steenson, L.R. and T.R. Klaenhammer. 1986. Plasmid heterogeneity in Streptococcus cremoris M12R: Effects on proteolytic activity and host dependent phage replication. J. Dairy Sci. 69:2227-2236.

28. Geis. A., H. and M. Teuber. 1987. Plasmid dependent bacteriophage resistance in lactic acid streptococci. Abstract from C.E.C. Meeting 'Culture Collections and Genetic Engineering of Microorganisms' Ioannia, Greece, pp. 33. Commission of the European Communities.

29. Davies, S.L. & M.G. Gasson, 1984. Bacteriophages of Dairy Lactic Acid Bacteria, in Advance in Microbiology and Biochemistry of Cheese and Fermented Milk. (Davies, F.L. & B.A. Law, eds.) pp127-151, Elsevier Applied Science Publishers, London.

TABLE 1: Conjugative transfer of Lac and phage insensitivity

| | | Lac$^+$ Transconjugants | |
|---|---|---|---|
| Donor | Recipient | Frequency/ Recipient | % phage 712 insensitive |
| | | | EG |
| S. Cremoris UC653 | S. lactis MG1363 Sm | $3.3 \times 10^{-7}$ | 70 (AB001) |
| S. lactis BA2 | S. lactis MG1363 Sm | $1.43 \times 10^{-4}$ | 11 (AC001) |
| S. lactis AB001 | S. lactis LM0230 | $7.6 \times 10^{-9}$ | 15 |
| S. lactis AC001 | S. lactis LM0230 | $1.8 \times 10^{-5}$ | 60 |

TABLE 2: Conjugative transfer of Lac and phage insensitivity

| Donor | Recipient | Lac$^+$ Transconjugants | | |
| | | Frequency/ Recipient | % phage 712 insensitive | Transconjugant Designation |
|---|---|---|---|---|
| S.lactis AB001 | S.lactis LM2306 | $4.0 \times 10^{-7}$ | 75.0 | |
| S.lactis AB002 | S.lactis LM2306 | $3.7 \times 10^{-3}$ | 91.7 | |
| S.cremoris UC653 | S.lactis MMS366 | $3.0 \times 10^{-7}$ | 100 | (a) MM20 |
| | | | | (b) MM21 |

18

TABLE 3

EFFECT OF TEMPERATURE ON REPLICATION OF SELECTED PHAGE ON S. LACTIS MG1363 SM AND BACTERIOPHAGE INSENSITIVE DERIVATIVES (EOP VALUES).

| STRAIN | S. LACTIS MG1363 SM | | | | | | S. LACTIS AC001 | | |
|---|---|---|---|---|---|---|---|---|---|
| TEMP  PHAGE | 712 | C2 | ML3 | EB1 | STL5 | SK1 | 712 | C2 | ML3 |
| 21°C | 1.0 | 1.0 | 1.0 | 1.0 | 1.0 | 1.0 | 0 | 0 | 0 |
| 30°C | 1.0 | 1.0 | 1.0 | 1.0 | 1.0 | 1.0 | 0 | $5 \times 10^{-2}$ | 1.0 |
| 37°C | ND | 1.0 | 1.0 | 1.0 | 1.0 | 1.0 | 0 | $1 \times 10^{-2}$ | 1.0 |
| 40°C | ND | 1.0 | 1.0 | ND | ND | ND | ND | $2 \times 10^{-1}$ | 1.0 |

| STRAIN | S. LACTIS AB001 | | | | | | S. LACTIS AC003 | | |
|---|---|---|---|---|---|---|---|---|---|
| TEMP  PHAGE | 712 | C2 | ML3 | EB1 | STL5 | SK1 | 712 | C2 | ML3 |
| 21°C | 0 | 0 | 0 | $3 \times 10^{-6}$ | $5 \times 10^{-3}$ | 0 | 0 | 0 | 0 |
| 30°C | 0 | $2 \times 10^{-2}$ | $5 \times 10^{-1}$ | $9 \times 10^{-3}$ | $8 \times 10^{-3}$ | 0 | 0 | $1 \times 10^{-7}$ | $2 \times 10^{-3}$ |
| 37°C | 0 | $2 \times 10^{-4}$ | $7 \times 10^{-1}$ | $6 \times 10^{-4}$ | $2 \times 10^{-3}$ | 0 | 0 | $1 \times 10^{-5}$ | $3 \times 10^{-1}$ |
| 40°C | ND | $7 \times 10^{-1}$ | $4 \times 10^{-1}$ | ND | ND | ND | ND | $9 \times 10^{-4}$ | $6 \times 10^{-1}$ |

EP 0 246 909 B1

TABLE 4    Transfer of the pCI750 and pCI829-associated phage insensitivity mechanism(s) to selected strains of lactic streptococci.

| Donor | Recipient | Lac$^+$ transconjugants | |
|---|---|---|---|
| | | Frequency/ Recipient | % insensitive to homo- logous phage |
| S. lactis AB002 | S. lactis SK3 Lac$^-$ Tc$^R$ | $2.0 \times 10^{-3}$ | 88 |
| | S. lactis subsp. diacetylactis 18-16 Lac$^-$ Tc$^R$ | $1.5 \times 10^{-4}$ | 50 |
| | S. cremoris UC411 Lac$^-$ Fus$^R$ | $1.5 \times 10^{-3}$ | 94 |
| S. lactis AC003 | S. lactis SK3 Lac$^-$ Tc$^R$ | $3.0 \times 10^{-3}$ | 83 |
| | S. lactis subsp. diacetylactis 18-16 Lac$^-$ Tc$^R$ | $5.0 \times 10^{-5}$ | 83 |
| | S. cremoris UC411 Lac$^-$ Fus$^R$ | $6.0 \times 10^{-4}$ | 50 |

TABLE 5    Data showing increased phage sensitivity of S. cremoris UC563 (ie. cured of the phage insensitivity plasmid pCI528) compared to the parent S. cremoris UC503 (containing pCI528).

| | Host | | | |
|---|---|---|---|---|
| | S. cremoris UC503 | | S. cremoris UC563 | |
| Phage | pfu/ml | plaque size (mm) | pfu/ml | plaque size (mm) |
| c2 | 0 | 0 | $3 \times 10^3$ | <1.55 |
| uc811 | 0 | 0 | $1 \times 10^2$ | <1 |

TABLE 6    Comparison of the sensitivity of S. lactis MG1363
           (plasmid-free) and S. lactis UC505 (containing the phage
           insensitivity plasmid pCI528) to selected virulent phage at
           30, 37 and 39°C.

| | pfu/ml at different temperatures (°C) | | | | | |
| | S. lactis MG1363 | | | S. lactis UC505 | | |
| Phage | 30 | 37 | 39 | 30 | 37 | 39 |
| 712 | $2 \times 10^9$ | $3 \times 10^8$ | $6 \times 10^8$ | 0 | 0 | 0 |
| jj50 | $2 \times 10^6$ | $2 \times 10^6$ | $3 \times 10^6$ | 0 | 0 | 0 |
| c2 | $4 \times 10^8$ | $7 \times 10^8$ | $3 \times 10^8$ | 0 | 0 | 0 |
| ml3 | $1 \times 10^{10}$ | $>10^{10}$ | $>10^{10}$ | 0 | 0 | 0 |

TABLE 7    Comparison of the efficiency of adsorption of phages to
           S. lactis MG1363 and S. lactis UC505 (containing pCI528).

| | % adsorption | |
| Phage | S. lactis MG1363 | S. lactis UC505 |
| 712 | 97.46 | 37.31 |
| jj50 | 94.91 | 40.98 |
| c2 | 94.53 | 45.34 |
| ml3 | 97.64 | 36 |

TABLE 8    Comparison of the ability of S. lactis MG1299 (containing pLP712) and S. lactis UC503 (containing pLP712 and pCI528) to reduce the pH of milk in the presence of phages 712, jj50, c2 and ml3 or a cocktail of all four using the Heap-Lawrence Milk Activity Test (13).

| Phage\cycle | S. lactis MG1299 | | | | | | S. lactis UC505 | | | | | |
| | control* | 712 | jj50 | c2 | ml3 | cocktail | control* | 712 | jj50 | c2 | ml3 | cocktail |
|---|---|---|---|---|---|---|---|---|---|---|---|---|
| 1 | 4.94 | 6.47 | 6.49 | 6.48 | 6.48 | 6.48 | 4.94 | 4.87 | 4.89 | 4.88 | 4.88 | 4.93 |
| 2 | 5.06 | 6.49 | 6.49 | 6.49 | 6.49 | 6.49 | 5.09 | 5.02 | 5.07 | 5.05 | 5.02 | 5.06 |
| 3 | 4.84 | 6.55 | 6.55 | 5.75 | 6.53 | 6.55 | 5.91 | 4.93 | 5.87 | 4.86 | 4.88 | 4.92 |
| 4 | 5.17 | 6.48 | 6.48 | 6.49 | 6.49 | 6.49 | 5.13 | 5.10 | 5.12 | 5.11 | 5.10 | 5.19 |
| 5 | 5.06 | 6.48 | 6.48 | 6.48 | 6.48 | 6.48 | 5.19 | 5.18 | 5.18 | 5.15 | 5.16 | 5.17 |
| 6 | 4.96 | 6.40 | 6.40 | 6.43 | 6.44 | 6.42 | 5.52 | 4.97 | 4.96 | 4.95 | 5.94 | 4.96 |
| 7 | 5.08 | 6.47 | 6.47 | 6.48 | 6.44 | 6.44 | 5.13 | 5.07 | 5.06 | 5.08 | 5.10 | 5.08 |
| 8 | 5.03 | 6.41 | 6.41 | 6.41 | 6.41 | 6.41 | 5.13 | 5.09 | 5.08 | 5.07 | 5.08 | 5.06 |
| 9 | 4.95 | 6.33 | 6.33 | 6.36 | 6.35 | 6.33 | 5.09 | 5.09 | 5.07 | 5.03 | 5.05 | 5.06 |
| 10 | 5.06 | 6.47 | 6.47 | 6.46 | 6.46 | 6.47 | 5.08 | 5.04 | 5.03 | 5.05 | 5.04 | 5.04 |
| 11 | 5.06 | 6.48 | 6.48 | 6.47 | 6.47 | 6.45 | 5.17 | 5.15 | 5.11 | 5.60 | 5.09 | 5.14 |
| 12 | 5.20 | 6.49 | 6.49 | 6.47 | 6.48 | 6.49 | 5.27 | 5.23 | 5.21 | 5.21 | 5.24 | 5.25 |

*   no phage added to the milk

0.1 ml of the appropriate phage suspension (each at approximately 10 pfu/ml) was added to cycle 1. In subsequent cycles (2-12) 0.5 ml of phage suspension and 0.05 of whey from the previous cycle were added.

Table 9      Effect of the pCI750 and pCI829 encoded mechanisms
             individually and together on the plaque size of phage
             712 and c2

| Host (plasmid) | plaque size of phage (mm) | |
| --- | --- | --- |
| | 712 | c2 |
| S. lactis MG1363 (plasmid-free) | 0 | 3 |
| S. lactis AB001 (pCI750) | 0 | 1-1.5 |
| S. lactis AC001 (pCI829) | 0 | 1-1.5 |
| S. lactis AC003 (pCI751 + pCI829) | 0 | 0 |

**Claims**
**Claims for the following Contracting States : AT, BE, CH, DE, FR, GB, GR, IT, LI, LU, NL, SE**

1. An isolated plasmid carrying one or more genes which are capable of conferring insensitivity to one or more phages upon Streptococcus cremoris bacteria, the resistance to such phages not being affected at temperatures of 35°C to 40°C, characterised in that the genes are genes carried in plasmids pCI750 pCI777 or pCI528, deposited in bacterial strains S. lactis AB001, AB002 and UC505 respectively at the National Collection of Industrial Bacteria, Aberdeen, Scotland under accession nos. 12261, 12262 and 12478 respectively, which are capable of also conferring phage insensitivity upon Streptococcus lactis or Streptococcus lactis subsp. diacetylactis, the capability of conferring phage insensitivity not being destroyed by temperatures of up to 39°C, or genes which have sufficient sequence homology therewith to also confer phage insensitivity upon S. lactis or S. lactis subsp. diacetylactis at temperatures of up to 39°C.

2. A plasmid as claimed in claim 1 originating in a Streptococcus cremoris strain.

3. A plasmid as claimed in claim 1 or claim 2 which confers insensitivity to both small isometric-headed and prolate-headed phage.

4. A recombinant plasmid as claimed in claim 1 or claim 3 carrying genes which encode phage insensitivity determinants and lactose utilization determinants in lactic streptococci.

5. A recombinant plasmid as claimed in claim 1 or claim 3 carrying genes which encode phage insensitivity determinants, lactose utilization determinants and high-frequency conjugative ability determinants in lactic streptococci.

6. A plasmid as claimed in any one of claims 1 to 5 selected from the group consisting of:-
   plasmid pCI750 as deposited in bacterial strain S. lactis AB001 at the National Collection of Industrial Bacteria, Aberdeen, Scotland under the accession no. 12261,
   plasmid pCI777 as deposited in bacterial strain S. lactis AB002 at the National Collection of Industrial Bacteria, Aberdeen, Scotland under accession no. 12262, or
   plasmid pCI528 as deposited in bacterial strain S. lactis UC505 at the National Collection of Industrial Bacteria, Aberdeen, Scotland, under accession no. 12478,
   plasmids carrying one or more of the phage insensitivity encoding genes of any of the said plasmids deposited at the NCIB under the said accession nos. 12261, 12262 and 12478,
   and plasmids having sufficient sequence homology with the said deposited plasmids to be capable of conferring phage insensitivity upon S. lactis or S. lactis subsp. diacetylactis as well as S. cremoris at temperatures of up to 39°C.

7. A plasmid as claimed in claim 1 having a molecular weight of 43.6 Mdal and the following restriction pattern:-

| Restriction Enzyme | Number of Fragments | Fragment Size (kb) |
|---|---|---|
| BamHI | 2 | 35.6, 30.4 |
| BclI | 7 | 21.32, 13.77, 12.55, 9.75, 4.78 2.13, 1.69 |
| Bg1II | Many | Not determined |
| BstEII | 2 | 56.74, 9.26 |
| EcoRV | 6 | 41.69, 11.1, 5.48, 5.26, 1.45, 1.08 |
| HindIII | 11 | 23.22, 12.18, 6.70, 5.08, 4.13, 3.51, 3.28, 2.43, 2.03, 1.85, 1.60 |
| HpaII | 10 | 20.97, 14.51, 10.26, 5.91, 3.35, 3.10, 2.25, 2.19, 1.70, 1.55 |
| PvuII | 5 | 26.01, 20.44, 9.66, 6.54, 8.35 |
| Sa1I | 4 | 25.33, 20.06, 13.16, 7.51 |

or a plasmid having a molecular weight of 27 Mdal and the following restriction pattern:-

| Restriction Enzyme | Number of Fragments | Fragment Size (kb) |
|---|---|---|
| SacI | 1 | 40.0 |
| StuI | 2 | 32.9, 6.9 |
| Bg1II | 3 | 20.8, 14.4, 4.0 |
| PvuII | 3 | 22.9, 8.3, 5.1 |
| ClaI | 4 | 21.2, 7.4, 6.1, 4.8 |
| EcoRV | 6 | 25.1, 5.9, 4.5, 3.1, 2.7, 2.1 |
| HaeIII | 4 | 25.1, 5.49, 5.0, 4.36 |
| HincII | 6 | 11.8, 5.6, 4.5, 3.8, 2.5, 1.7 |
| AvaI | 5 | 16.3, 12.5, 9.1, 4.6, 3.2 |
| HpaII | 8 | 12.6, 11.9, 10.8, 3.9, 2.4, 2.2, 1.2, 0.4 |
| EcoRI | 8 | 21.0, 12.1, 2.5, 2.1, 1.7, 0.84, 0.69, 0.41 |

8. A DNA sequence of a plasmid as claimed in claim 6 or 7 encoding phage insensitivity determinants or a DNA having a sufficient sequence homology therewith to also confer phage insensitivity upon S. lactis or S. lactis subsp. diacetylactis as well as S. cremoris bacteria at temperatures of up to 39°C.

9. A bacterial host containing one or more plasmids as claimed in any of claims 1 to 7, not being a naturally occurring host/plasmid combination.

10. A bacterial host as claimed in claim 9 which is a lactic streptococcal host.

11. A culture selected from dairy starter cultures, cheese starter cultures, meat and meat fermentation cultures, vegetable fermentation cultures or probiotic cultures containing a plasmid as claimed in any of claims 1 to 7 or a bacterial host as claimed in either of claims 9 and 10.

12. A culture as claimed in claim 11 in liquid, frozen, freeze-dried, concentrated frozen or concentrated freeze-dried form.

13. A method of conferring phage insensitivity on a cheese-making bacterium comprising introducing into the bacterium a plasmid as claimed in any of claims 1 to 7, so as to provide other than a naturally occurring bacterial host/plasmid combination.

14. A method of conferring phage insensitivity on a food starter culture comprising bacteria wherein a plasmid according to any of claims 1 to 7 is introduced into the said bacteria, so as to provide other than a naturally occurring bacterial host/plasmid combination.

**Claims for the following Contracting State : ES**

1. A method of preparing an isolated plasmid carrying one or more genes which are capable of conferring insensitivity to one or more phages upon Streptococcus cremoris bacteria, the resistance to such phages not being affected at temperatures of 35°C to 40°C, characterised in that the genes are genes carried in plasmids pCI750, pCI777 or pCI528, deposited in bacterial strains S. lactis AB001, AB002 and UC505 respectively at the National Collection of Industrial Bacteria, Aberdeen, Scotland under accession nos. 12261, 12262 and 12478 respectively, which are capable of also conferring phage insensitivity upon Streptococcus lactis or Streptococcus lactis subsp. diacetylactis, the capability of conferring phage insensitivity not being destroyed by temperatures of up to 39°C, or genes which have sufficient sequence homology therewith to also confer phage insensitivity upon S. lactis or S. lactis subsp. diacetylactis at temperatures of up to 39°C and that the plasmid is isolated from the original host.

2. A method as claimed in claim 1 wherein the original plasmid originates from a Streptococcus cremoris strain.

3. A method as claimed in claim 1 or claim 2 in which the plasmid confers insensitivity to both small isometric-headed and prolate-headed phage.

4. A method as claimed in claim 1 or claim 3 in which the plasmid is recombinant and carries genes which encode phage insensitivity determinants and lactose utilization determinants in lactic streptococci.

5. A method as claimed in claim 1 or claim 3 in which the plasmid is recombinant and carries genes which encode phage insensitivity determinants, lactose utilization determinants and high-frequency conjugative ability determinants in lactic streptococci.

6. A method as claimed in any one of claims 1 to 5 in which the plasmid is selected from the group consisting of:-

   plasmid pCI750 as deposited in bacterial strain S. lactis AB001 at the National Collection of Industrial Bacteria, Aberdeen, Scotland under the accession no. 12261,

   plasmid pCI777 as deposited in bacterial strain S. lactis AB002 at the National Collection of Industrial Bacteria, Aberdeen, Scotland under accession no. 12262, or

   plasmid pCI528 as deposited in bacterial strain S. lactis UC505 at the National Collection of Industrial Bacteria, Aberdeen, Scotland, under accession no. 12478,

   plasmids carrying one or more of the phage insensitivity encoding genes of any of the said plasmids deposited at the NCIB under the said accession nos. 12261, 12262 and 12478,

   and plasmids having sufficient sequence homology with the said deposited plasmids to be capable of conferring phage insensitivity upon S. lactis or S. lactis subsp. diacetylactis as well as S. cremoris at temperatures of up to 39°C.

7. A method as claimed in claim 1 in which the plasmid has a molecular weight of 43.6 Mdal and the following restriction pattern:-

| Restriction Enzyme | Number of Fragments | Fragment Size (kb) |
|---|---|---|
| BamHI | 2 | 35.6, 30.4 |
| BcII | 7 | 21.32, 13.77, 12.55, 9.75, 4.78 2.13, 1.69 |
| Bg1II | Many | Not determined |
| BstEII | 2 | 56.74, 9.26 |
| EcoRV | 6 | 41.69, 11.1, 5.48, 5.26, 1.45, 1.08 |
| HindIII | 11 | 23.22, 12.18, 6.70, 5.08, 4.13, 3.51, 3.28, 2.43, 2.03, 1.85, 1.60 |
| HpaII | 10 | 20.97, 14.51, 10.26, 5.91, 3.35, 3.10, 2.25, 2.19, 1.70, 1.55 |
| PvuII | 5 | 26.01, 20.44, 9.66, 6.54, 8.35 |
| Sa1I | 4 | 25.33, 20.06, 13.16, 7.51 |

or a molecular weight of 27 Mdal and the following restriction pattern:-

| Restriction Enzyme | Number of Fragments | Fragment Size (kb) |
|---|---|---|
| SacI | 1 | 40.0 |
| StuI | 2 | 32.9, 6.9 |
| Bg1II | 3 | 20.8, 14.4, 4.0 |
| PvuII | 3 | 22.9, 8.3, 5.1 |
| ClaI | 4 | 21.2, 7.4, 6.1, 4.8 |
| EcoRV | 6 | 25.1, 5.9, 4.5, 3.1, 2.7, 2.1 |
| HaeIII | 4 | 25.1, 5.49, 5.0, 4.36 |
| HincII | 6 | 11.8, 5.6, 4.5, 3.8, 2.5, 1.7 |
| AvaI | 5 | 16.3, 12.5, 9.1, 4.6, 3.2 |
| HpaII | 8 | 12.6, 11.9, 10.8, 3.9, 2.4, 2.2, 1.2, 0.4 |
| EcoRI | 8 | 21.0, 12.1, 2.5, 2.1, 1.7, 0.84, 0.69, 0.41 |

8.  A method of preparing a DNA sequence in which a plasmid isolated by a method as claimed in claim 6 or 7 is selected and DNA encoding phage insensitivity determinants or DNA having a sufficient sequence homology therewith to also confer phage insensitivity upon S. lactis or S. lactis subsp. diacetylactis as well as S. cremoris bacteria at temperatures of up to 39°C is isolated.

9.  A method of preparing a bacterial host in which one or more plasmids isolated by a method as claimed in any one of claims 1 to 7, is inserted into a bacterium, the host/plasmid combination not being a naturally occurring combination.

10.  A method as claimed in claim 9 in which the host is a lactic streptococcal host.

11.  A method of conferring phage insensitivity on a cheese-making bacterium comprising introducing into the bacterium a plasmid isolated according to a method as claimed in any one of claims 1 to 7, so as to provide other than a naturally occurring bacterial host/plasmid combination.

12.  A method of conferring phage insensitivity on a food starter culture comprising bacteria wherein a plasmid isolated according to a method of any one of claims 1 to 7 is introduced into the said bacteria, so as to provide other than a naturally occurring bacterial host/plasmid combination.

13.  A method as claimed in claim 12 wherein the starter culture is selected from dairy starter cultures, cheese starter cultures, meat and meat fermentation cultures, vegetable fermentation cultures or probiotic cultures.

14.  A method as claimed in claim 13 wherein the culture is stored in liquid, frozen, freeze-dried, concentrated frozen or concentrated freeze-dried form.

**Patentansprüche**

**Patentansprüche für folgende Vertragsstaaten : AT, BE, CH, DE, FR, GB, FR, IT, LI, LU, NL, SE**

1.  Isoliertes Plasmid, welches ein Gen oder mehrere Gene trägt, das/die Unempfindlichkeit gegenüber einem oder mehreren Phagen auf Streptococcus cremoris-Bakterien übertragen kann/können, wobei die Resistenz gegenüber solchen Phagen bei Temperaturen von 35 bis 40°C nicht beeinträchtigt wird, dadurch gekennzeichnet, daß die Gene Gene sind, welche in den Plasmiden pCI750, pCI777 oder pCI528 vorhanden sind, hinterlegt in den Bakterienstämmen S. lactis AB001, AB002 und UC505 bei der National Collection of Industrial Bacteria, Aberdeen, Schottland, unter den Hinterlegungsnummern 12261, 12262 und 12478, die Phasgus-Unempfindlichkeit auch auf Streptococcus lactis oder Streptococcus lactis subsp. diacetylactis übertragen können, wobei die Fähigkeit, Phagus-Unempfindlichkeit zu übertragen bzw. zu verleihen, nicht durch Temperaturen bis zu 39°C zerstört wird, oder Gene, die ausreichende Sequenzhomologie damit besitzen, um ebenfalls Phagus-Unempfindlichkeit auf S. lactis oder S. lactis subsp. diacetylactis bei Temperaturen bis zu 39°C zu übertragen.

2.  Plasmid nach Anspruch 1, das seinen Ursprung in einem Streptococcus cremoris-Stamm hat.

3.  Plasmid nach Anspruch 1 oder 2, das Unempfindlichkeit gegenüber kleinen Phagen mit sowohl isometrischem Kopf als auch mit Prolat-Kopf verleiht.

4.  Rekombinantes Plasmid nach Anspruch 1 oder 3, welches Gene trägt, die Determinanten für Phagus-Unempfindlichkeit und Determinanten für Lactoseverwertung in Milch-Streptokokken codieren.

5.  Rekombinantes Plasmid nach Anspruch 1 oder 3, welches Gene trägt, die Determinanten für Unempfindlichkeit, Determinanten für Lactoseverwertung und Determinanten für die Fähigkeit zur Hochfrequenz-Konjugation in Milch-Streptokokken codieren.

6.  Plasmid nach einem der Ansprüche 1 bis 5, ausgewählt aus der Gruppe bestehend aus:
    Plasmid pCI750, hinterlegt im Bakterienstamm S. lactis AB001 bei der National Collection of Industrial Bacteria, Aberdeen, Schottland, unter der Hinterlegungsnummer 12261,
    Plasmid pCI777, hinterlegt im Bakterienstamm S. lactis AB002 bei der National Collection of Industrial Bacteria, Aberdeen, Schottland, unter der Hinterlegungsnummer 12262, oder
    Plasmid pCI528, hinterlegt im Bakterienstamm S. lactis UC505 bei der National Collection of Industrial Bacteria, Aberdeen, Schottland, unter der Hinterlegungsnummer 12478,
    Plasmide, die ein oder mehrere der Phagus-Unempfindlichkeit codierenden Gene eines der genannten, bei der NCIB unter den genannten Hinterlegungsnummern 12261, 12262 und 12478 hinterlegten Plasmide tragen,
    sowie Plasmide, die ausreichende Sequenzhomologie mit den genannten hinterlegten Plasmiden aufweisen, um S. lactis oder S. lactis subsp. diacetylactis sowie S. cremoris bei Temperaturen bis zu 39°C Phagus-Unempfindlichkeit verleihen zu können.

7.  Plasmid nach Anspruch 1 mit einem Molekulargewicht von 43,6 Mdal und dem folgenden Restriktionsmuster:

| Restriktions-Enzym | Anzahl der Fragmente | Fragmentgröße (kb) |
|---|---|---|
| BamHI | 2 | 35,6, 30,4 |
| BclI | 7 | 21,32, 13,77, 12,55, 9,75, 4,78 2,13, 1,69 |
| Bg1II | viele | nicht bestimmt |
| BstEII | 2 | 56,74, 9,26 |
| EcoRV | 6 | 41,69, 11,1, 5,48, 5,26, 1,45, 1,08 |
| HindIII | 11 | 23,22, 12,18, 6,70, 5,08, 4,13, 3,51, 3,28, 2,43, 2,03, 1,85, 1,60 |
| HpaII | 10 | 20,97, 14,51, 10,26, 5,91, 3,35, 3,10, 2,25, 2,19, 1,70, 1,55 |
| PvuII | 5 | 26,01, 20,44, 9,66, 6,54, 8,35 |
| Sa1I | 4 | 25,33, 20,06, 13,16, 7,51 |

oder Plasmid mit einem Molekulargewicht von 27 Mdal und dem folgenden Restriktionsmuster:

EP 0 246 909 B1

| Restriktions-Enzym | Anzahl der Fragmente | Fragmentgröße (kb) |
|---|---|---|
| SacI | 1 | 40,0 |
| StuI | 2 | 32,9, 6,9 |
| Bg1II | 3 | 20,8, 14,4, 4,0 |
| PvuII | 3 | 22,9, 8,3, 5,1 |
| ClaI | 4 | 21,2, 7,4, 6,1, 4,8 |
| EcoRV | 6 | 25,1, 5,9, 4,5, 3,1, 2,7, 2,1 |
| HaeIII | 4 | 25,1, 5,49, 5,0, 4,36 |
| HincII | 6 | 11,8, 5,6, 4,5, 3,8, 2,5, 1,7 |
| AvaI | 5 | 16,3, 12,5, 9,1, 4,6, 3,2 |
| HpaII | 8 | 12,6, 11,9, 10,8, 3,9, 2,4, 2,2, 1,2, 0,4 |
| EcoRI | 8 | 21,0, 12,1, 2,5, 2,1, 1,7, 0,84, 0,69, 0,41 |

8. DNA-Sequenz eines Plasmids nach Anspruch 6 oder 7, die Determinanten für Phagus-Unempfindlichkeit codiert, oder DNA mit ausreichender Sequenzhomologie dazu, um ebenfalls S. lactis oder S. lactis subsp. diacetylactis sowie S. cremoris-Baterien bei Temperaturen bis zu 39°C Phagus-Unempfindlichkeit zu verleihen.

9. Bakterieller Wirt, der ein oder mehrere Plasmide nach einem der Ansprüche 1 bis 7 enthält, wobei dies nicht eine natürlich auftretende Wirt/Plasmid-Kombination ist.

10. Bakterieller Wirt nach Anspruch 9, der ein Milch-Streptococcus-Wirt ist.

11. Kultur, ausgewählt aus Molkerei-Starterkulturen, Käse-Starterkulturen, Fleisch- und Fleisch-Fermentationskulturen, Pflanzen-Fermentationskulturen oder probiotischen Kulturen, enthaltend ein Plasmid nach einem der Ansprüche 1 bis 7, oder einen bakterieller Wirt nach einem der Ansprüche 9 und 10.

12. Kultur nach Anspruch 11 in flüssiger, tiefgefrorener, gefriergetrockneter, konzentrierter tiefgefrorener oder konzentrierter gefriergetrockneter Form.

13. Verfahren zum Übertragen von Phagus-Unempfindlichkeit auf ein Käse-herstellendes Bakterium, dadurch gekennzeichnet, daß man in das Bakterium ein Plasmid nach einem der Ansprüche 1 bis 7 einführt, um eine andere als eine natürlich, auftretende Kombination bakterieller Wirt/Plasmid bereitzustellen,

14. Verfahren zum Übertragen von Phagus-Unempfindlichkeit auf eine Lebensmittel-Starterkultur, welche Bakterien enthält, in denen ein Plasmid nach einem der Ansprüche 1 bis 7 in die Bakterien eingeführt worden ist, um eine andere als natürlich auftretende Kombination bakterieller Wirt/Plasmid bereitzustel-len.

**Patentansprüche für folgenden Vertragsstaat : ES**

1. Verfahren zur Herstellung eines isolierten Plasmids, welches ein Gen, oder mehrere Gene trägt, das/die Unempfindlichkeit gegenüber einem oder mehreren Phagen auf Streptococcus cremoris-Bakterien übertragen kann/ können, wobei die Resistenz gegenüber solchen Phagen bei Temperaturen von 35 bis 40°C nicht beeinträchtigt wird, dadurch gekennzeichnet, daß die Gene Gene sind, welche in den Plasmiden pCI750, pCI777 oder pCI528 vorhanden sind, hinterlegt in den Bakterienstämmen S. lactis AB001, AB002 und UC505 bei der National Collection of Industrial Bacteria, Aberdeen, Schottland, unter den Hinterlegungsnummern 12261, 12262 und 12478, die Phasgus-Unempfindlichkeit auch auf Streptococcus lactis oder Streptococcus lactis subsp. diacetylactis übertragen können, wobei die Fähigkeit, Phagus-Unempfindlichkeit zu übertragen bzw. zu verleihen, nicht durch Temperaturen bis zu 39°C zerstört wird, oder Gene, die ausreichende Sequenzhomologie damit besitzen, um ebenfalls Phagus-Unempfindlichkeit auf S. lactis oder S. lactis subsp. diacetylactis bei Temperaturen bis zu 39°C zu übertragen, und daß das Plasmid aus dem ursprünglichen Wirt isoliert wird.

28

**2.** Verfahren nach Anspruch 1, dadurch gekennzeichnet, daß das Plasmid seinen Ursprung in einem Streptococcus cremoris-Stamm hat.

**3.** Verfahren nach Anspruch 1 oder 2, dadurch gekennzeichnet, daß das Plasmid Unempfindlichkeit gegenüber kleinen Phagen mit sowohl isometrischem Kopf als auch mit Prolat-Kopf verleiht.

**4.** Verfahren nach Anspruch 1 oder 3, dadurch gekennzeichnet, daß das Plasmid rekombinant ist und Gene trägt, die Determinanten für Phagus-Unempfindlichkeit und Determinanten für Lactoseverwertung in Milch-Streptokokken codieren.

**5.** Verfahren nach Anspruch 1 oder 3, dadurch gekennzeichnet, daß das Plasmid rekombinant ist und Gene trägt, die Determinanten für Unempfindlichkeit, Determinanten für Lactoseverwertung und Determinanten für die Fähigkeit zur Hochfrequenz-Konjugation in Milch-Stroptokokken codieren.

**6.** Verfahren nach einem der Ansprüche 1 bis 3, dadurch gekennzeichnet, daß das Plasmid ausgewählt wird aus der Gruppe bestehend aus:

Plasmid pCI750, hinterlegt im Bakterienstamm S. lactis AB001 bei der National Collection of Industrial Bacteria, Aberdeen, Schottland, unter der Hinterlegungsnummer 12261,

Plasmid pCI777, hinterlegt im Bakterienstamm S. lactis AB002 bei der National Collection of Industrial Bacteria, Aberdeen, Schottland, unter der Hinterlegungsnummer 12262, oder

Plasmid pCI528, hinterlegt im Bakterienstamm S. lactis UC505 bei der National Collection of Industrial Bacteria, Aberdeen, Schottland, unter der, Hinterlegungsnummer 12478,

Plasmide, die ein oder mehrere der Phagus-Unempfindlichkeit codierenden Gene eines der genannten, bei der NCIB unter den genannten Hinterlegungsnummern 12261, 12262 und 12470 hinterlegten Plasmide tragen,

sowie Plasmide, die ausreichende Sequenzhomologiemit den genannten hinterlegten Plasmiden aufweisen, um S. lactis oder S. lactis subsp. diacetylactis sowie S. cremoris bei Temperaturen bis zu 39°C Phagus-Unempfindlichkeit verleihen zu können.

**7.** Verfahren nach Anspruch, 1 dadurch gekennzeichnet, daß das Plasmid ein Molokulargewicht von 43,6 Mdal und das folgende Restriktionsmuster aufweist:

| Restriktions-Enzym | Anzahl der Fragmente | Fragmentgröße (kb) |
|---|---|---|
| BamII | 2 | 35,6, 30,4 |
| BcII | 7 | 21,32, 13,77, 12,55, 9,75, 4,78 2,13, 1.69 |
| Bg1II | viele | nicht bestimmt |
| BstEII | 2 | 56.74, 9,26 |
| EcoRV | 6 | 41,69, 11,1, 5,48, 5,26, 1,45, 1,08 |
| HindIII | 11 | 23,22, 12,18, 6,70, 5,08 4,13, 3,51, 3,28, 2,43, 2,03, 1,85, 1,60 |
| HpaII | 10 | 20,97, 14,51, 10,26, 5,91, 3,35, 3,10, 2,25, 2,19, 1,70, 1,55 |
| PvuII | 5 | 26,01, 20,44, 9,66, 6,54, 8,35 |
| Sa1I | 4 | 25,33, 20,06, 13,16, 7,51 |

oder ein Molekulargewicht von 27 Mdal und das folgende Restriktionsmuster:

| Restriktions-Enzym | Anzahl der Fragmente | Fragmentgröße (kb) |
|---|---|---|
| SacI | 1 | 40,0 |
| StuI | 2 | 32,9, 6,9 |
| Bg1II | 3 | 20,8, 14,4, 4,0 |
| PvuII | 3 | 22,9, 8,3, 5,1 |
| ClaI | 4 | 21,2, 7,4, 6,1, 4,8 |
| EcoRV | 6 | 25,1, 5,9, 4,5, 3,1, 2,7, 2,1 |
| HaeIII | 4 | 25,1, 5,49, 5,0 4,36 |
| HincII | 6 | 11,8, 5,6, 4,5, 3,8, 2,5, 1,7 |
| AvaI | 5 | 16,3, 12,5, 9,1, 4,6, 3,2 |
| HpaII | 8 | 12,6, 11,9, 10,8, 3,9, 2,4, 2,2, 1,2 0,4 |
| EcoRI | 8 | 21,0, 12,1, 2,5, 2,1, 1,7, 0,84, 0,69, 0,41 |

8. Verfahren zur Herstellung einer DNA-Sequenz, dadurch gekennzeichnet, daß ein Plasmid, isoliert durch ein Verfahren nach Anspruch 6 oder 7, ausgewählt wird und DNA, die Determinanten für Phagus-Unempfindlichkeit codiert, oder DNA mit ausreichender Sequenzhomologie dazu, um ebenfalls S. lactis oder S. lactis subsp. diacetylactis sowie S. cremoris-Baterien bei Temperaturen bis zu 39°C Phagus-Unempfindlichkeit zu verleihen, isoliert wird.

9. Verfahren zur Herstellung eines bakteriellen Wirts, dadurch gekennzeichnet, daß ein oder mehrere Plasmide, isoliert gemäß einem Verfahren nach einem der Ansprüche 1 bis 7, in ein Bakterium insertiert wird/werden, wobei die Wirt/Plasmid-Kombination keine natürlich auftretende Kombination ist.

10. Verfahren nach Anspruch 9, dadurch gekennzeichnet, daß der bakterielle Wirt ein Milch-Streptococcus-Wirt ist.

11. Verfahren zum Übertragen von Phagus-Unempfindlichkeit auf ein Käse-herstellendes Bakterium, dadurch gekennzeichnet, daß man in das Bakterium ein Plasmid isoliert nach einem Verfahren nach einem der Ansprüche 1 bis 7 einführt, um eine andere als eine natürlich auftretende Kombination bakterieller Wirt/Plasmid bereitzustellen.

12. Verfahren zum Übertragen von Phagus-Unempfindlichkeit auf eine Lebensmittel-Starterkultur, welche Bakterien enthält, dadurch gekennzeichnet, daß ein Plasmid nach einem der Ansprüche 1 bis 7 in die Bakterien eingeführt wird, um eine andere als natürlich auftretende Kombination bakterieller Wirt/Plasmid bereitzustellen.

13. Verfahren nach Anspruch 12, dadurch gekennzeichnet, daß die Starterkultur aus Molkerei-Starterkulturen, Käse-Starterkulturen, Fleisch- und Fleisch-Fermentationskulturen, Pflanzen-Fermentationskulturen oder probiotischen Kulturen ausgewählt wird.

14. Verfahren nach Anspruch 13, dadurch gekennzeichnet, daß die Kultur in flüssiger, tiefgefrorener, gefriergetrockneter, konzentrierter tiefgefrorener oder konzentrierter gefriergetrockneter Form gelagert wird.

**Revendications**

**Revendications pour les Etats contractants suivants : AT, BE, CH, DE, FR, GB, GR, IT, LI, LU, NL, SE**

1. Plasmide isolé portant un ou plusieurs gènes qui sont capables de conférer une insensibilité vis-à-vis d'un ou plusieurs phages à des bactéries de l'espèce Streptococcus cremoris, la résistance à ces phages n'étant pas modifiée à des températures de 35°C à 40°C, caractérisé en ce que les gènes sont des gènes portés par les plasmides pCI750, pCI777 ou pCI528, déposes dans les souches bactériennes S. lactis AB001, AB002 et UC505, respectivement, dans la National Collection of Industrial Bacteria, Aberdeen, Ecosse, sous les numéros de dépôt respectifs 12261, 12262 et 12478, qui sont capables également de conférer une insensibilité vis-à-vis de phages à Streptococcus lactis ou Streptococcus lactis sous-espèce diacetylactis, l'aptitude à conférer une insensibilité vis-à-vis de

30

phages n'étant pas détruite par des températures allant jusqu'à 39°C, ou des gènes qui possèdent une homologie de séquence suffisante avec ces gènes pour conférer également une insensibilité vis-à-vis de phages à S. lactis ou S. lactis sous-espèce diacetylactis à des températures allant jusqu'à 39°C.

2. Plasmide suivant la revendication 1, provenant d'une souche de Streptococcus cremoris.

3. Plasmide suivant la revendication 1 ou la revendication 2, qui confère une insensibilité à de petits phages à tête isométrique et à tête allongée.

4. Plasmide recombinant suivant la revendication 1 ou la revendication 3, portant des gènes qui codent pour des déterminants d'insensibilité vis-à-vis de phages et des déterminants d'utilisation du lactose dans des streptocoques lactiques.

5. Plasmide recombinant suivant la revendication 1 ou la revendication 3, qui porte des gènes qui codent pour des déterminants d'insensibilité vis-à-vis de phages, des déterminants d'utilisation du lactose et des déterminants d'aptitude à la conjugaison à haute fréquence dans des streptocoques lactiques.

6. Plasmide suivant l'une quelconque des revendications 1 à 5, choisi dans le groupe consistant en :
le plasmide pCI750 déposé dans la souche bactérienne S. lactis AB001 à la National Collection of Industrial Bacteria, Aberdeen, Ecosse, sous le numéro de dépôt 12261,
le plasmide pCI777 déposé dans la souche bactérienne S. lactis AB002 à la National Collection of Industrial Bacteria, Aberdeen, Ecosse, sous le numéro de dépôt 12262,
le plasmide pCI528 déposé dans la souche bactérienne S. lactis UC505 à la National Collection of Industrial Bacteria, Aberdeen, Ecosse, sous le numéro de dépôt 12478,
des plasmides portant un ou plusieurs des gènes, codant pour l'insensibilité vis-à-vis de phages, de l'un quelconque desdits plasmides déposés à la NCIB sous les numéros de dépôt 12261, 12262 et 12478,
et des plasmides présentant une homologie de séquence suffisante avec lesdits plasmides déposés pour être capables de conférer une insensibilité vis-à-vis de phages à S. lactis ou S. lactis sous-espèce diacetylactis ainsi qu'à S. cremoris à des températures allant jusqu'à 39°C.

7. Plasmide suivant la revendication 1, ayant un poids moléculaire de 43,6 Mdal et le schéma de restriction suivant :

| Enzyme de restriction | Nombre de fragments | Dimensions des fragments (kb) |
|---|---|---|
| BamHI | 2 | 35,6, 30,4 |
| BclI | 7 | 21,32, 13,77, 12,55, 9,75, 4,78, 2,13, 1,69 |
| BglII | Elevé | Non déterminées |
| BstEII | 2 | 56,74, 9,26 |
| EcoRV | 6 | 41,69, 11,1, 5,48, 5,26, 1,45, 1,08 |
| HindIII | 11 | 23,22, 12,18, 6,70, 5,08, 4,13, 3,51, 3,28, 2,43, 2,03, 1,85, 1,60 |
| HpaII | 10 | 20,97, 14,51, 10,26, 5,91, 3,35, 3,10, 2,25, 2,19, 1,70, 1,55 |
| PvuII | 5 | 26,01, 20,44, 9,66, 6,54, 8,35 |
| SalI | 4 | 25,33, 20,06, 13,16, 7,51 |

ou un plasmide ayant un poids moléculaire de 27 Mdal et le schéma de restriction suivant :

| Enzyme de restriction | Nombre de fragments | Dimensions des fragments (kb) |
|---|---|---|
| SacI | 1 | 40,0 |
| StuI | 2 | 32,9, 6,9 |
| BglII | 3 | 20,8, 14,4, 4,0 |
| PvuII | 3 | 22,9, 8,3, 5,1 |
| ClaI | 4 | 21,2, 7,4, 6,1, 4,8 |
| EcoRV | 6 | 25,1, 5,9, 4,5, 3,1, 2,7, 2,1 |
| HaeIII | 4 | 25,1, 5,49, 5,0, 4,36 |
| HincII | 6 | 11,8, 5,6, 4,5, 3,8, 2,5, 1,7 |
| AvaI | 5 | 16,3, 12,5, 9,1, 4,6, 3,2 |
| HpaII | 8 | 12,6, 11,9, 10,8, 3,9, 2,4, 2,2, 1,2, 0,4 |
| EcoRI | 8 | 21,0, 12,1, 2,5, 2,1, 1,7, 0,84, 0,69, 0,41 |

8. Séquence d'ADN d'un plasmide suivant la revendication 6 ou 7 codant pour des déterminants d'insensibilité vis-à-vis de phages, ou ADN présentant une homologie de séquence suffisante avec cette séquence d'ADN pour conférer également une insensibilité vis-à-vis de phages à des bactéries appartenant à S. lactis ou S. lactis sous-espèce diacetylactis ainsi qu'à S. cremoris à des températures allant jusqu'à 39°C.

9. Hôte bactérien contenant un ou plusieurs plasmides suivant l'une quelconque des revendications 1 à 7, ne consistant pas en une association hôte/plasmide naturelle.

10. Hôte bactérien suivant la revendication 9, qui est un hôte consistant en un streptocoque lactique.

11. Culture choisie entre des levains pour produits laitiers, des levains pour fromages, des cultures de viande et de fermentation de viande, des cultures de fermentation de légumes ou des cultures probiotiques contenant un plasmide suivant l'une quelconque des revendications 1 à 7 ou un hôte bactérien suivant chacune des revendications 9 et 10.

12. Culture suivant la revendication 11, sous forme liquide, congelée, lyophilisée, congelée concentrée ou lyophilisée concentrée.

13. Procédé pour conférer une insensibilité vis-à-vis de phages à une bactérie utilisée pour la production du fromage, comprenant l'introduction dans la bactérie d'un plasmide suivant l'une quelconque des revendications 1 à 7, de manière à fournir une association hôte bactérien/plasmide autre qu'une association hôte bactérien/plasmide naturelle.

14. Procédé pour conférer une insensibilité vis-à-vis de phages à une culture de départ pour produit alimentaire comprenant des bactéries, dans lequel un plasmide suivant l'une quelconque des revendications 1 à 7 est introduit dans lesdites bactéries, de manière à fournir une association hôte bactérien/plasmide autre qu'une association hôte bactérien/plasmide naturelle.

**Revendications pour l'Etat contractant suivant : ES**

1. Procédé de préparation d'un plasmide isolé portant un ou plusieurs gènes qui sont capables de conférer une insensibilité vis-à-vis d'un ou plusieurs phages à des bactéries de l'espèce Streptococcus cremoris, la résistance à ces phages n'étant pas modifiée à des températures de 35°C à 40°C, caractérisé en ce que les gènes sont des gènes portés par les plasmides pCl750, pCl777 ou pCl528, déposés dans les souches bactériennes S. lactis AB001, AB002 et UC505, respectivement, à la National Collection of Industrial Bacteria, Aberdeen, Ecosse, sous les numéros de dépôt 12261, 12262 et 12478, respectivement, qui sont capables également de conférer une insensibilité vis-à-vis de phages à Streptococcus lactis ou Streptococcus lactis sous-espèce diacetylactis, l'aptitude à conférer une insensibilité vis-à-vis de phages n'étant pas détruite par des températures allant jusqu'à 39°C, ou des gènes qui présentent une homologie de séquence suffisante avec ces gènes pour conférer également une insensibilité vis-à-vis de phages à S. lactis ou S. lactis sous-espèce diacetylactis à des températures allant jusqu'à 39 ° C, et en ce que le plasmide est isolé de l'hôte initial.

2. Procédé suivant la revendication 1, dans lequel le plasmide initial provient d'une souche de Streptococcus cremoris.

3. Procédé suivant la revendication 1 ou la revendication 2, dans lequel le plasmide confère une insensibilité vis-à-vis de petits phages à tête isométrique et à tête allongée.

4. Procédé suivant la revendication 1 ou la revendication 3, dans lequel le plasmide est recombinant et porte des gènes qui codent pour des déterminants d'insensibilité vis-à-vis de phages et des déterminants d'utilisation du lactose dans des streptocoques lactiques.

5. Procédé suivant la revendication 1 ou la revendication 3, dans lequel le plasmide est recombinant et porte des gènes qui codent pour des déterminants d'insensibilité vis-à-vis de phages, des déterminants d'utilisation du lactose et des déterminants d'aptitude à la conjugaison à haute fréquence dans des streptocoques lactiques.

6. Procédé suivant l'une quelconque des revendications 1 à 5, dans lequel le plasmide est choisi dans le groupe consistant en :

le plasmide pCI750 déposé dans la souche bactérienne S. lactis AB001 à la National Collection of Industrial Bacteria, Aberdeen, Ecosse, sous le numéro de dépôt 12261,

le plasmide pCI777 déposé dans la souche bactérienne S. lactis AB002 à la National Collection of Industrial Bacteria, Aberdeen, Ecosse, sous le numéro de dépôt 12262,

le plasmide pCI528 déposé dans la souche bactérienne S. lactis UC505 à la National Collection of Industrial Bacteria, Aberdeen, Ecosse, sous le numéro de dépôt 12478,

des plasmides portant un ou plusieurs des gènes codant pour l'insensibilité vis-à-vis de phages de l'un quelconque desdits plasmides déposés à la NCIB sous lesdits numéros de dépôt 12261, 12262 et 12478,

et des plasmides présentant une homologie de séquence suffisante avec lesdits plasmides déposés pour être capables de conférer une insensibilité vis-à-vis de phages à S. lactis ou S. lactis sous-espèce diacetylactis ainsi qu'à S. cremoris à des températures allant jusqu'à 39 ° C.

7. Procédé suivant la revendication 1, dans lequel le plasmide possède un poids moléculaire de 43,6 Mdal et le schéma de restriction suivant :

| Enzyme de restriction | Nombre de fragments | Dimensions des fragments (kb) |
|---|---|---|
| BamHI | 2 | 35,6, 30,4 |
| BclI | 7 | 21,32, 13,77, 12,55, 9,75, 4,78, 2,13, 1,69 |
| BglII | Elevé | Non déterminées |
| BstEII | 2 | 56,74, 9,26 |
| EcoRV | 6 | 41,69, 11,1, 5,48, 5,26, 1,45, 1,08 |
| HindIII | 11 | 23,22, 12,18, 6,70, 5,08, 4,13, 3,51, 3,28, 2,43, 2,03, 1,85, 1,60 |
| HpaII | 10 | 20,97, 14,51, 10,26, 5,91, 3,35, 3,10, 2,25, 2,19, 1,70, 1,55 |
| PvuII | 5 | 26,01, 20,44, 9,66, 6,54, 8,35 |
| SalI | 4 | 25,33, 20,06, 13,16, 7,51 |

ou un plasmide ayant un poids moléculaire de 27 Mdal et le schéma de restriction suivant :

| Enzyme de restriction | Nombre de fragments | Dimensions des fragments (kb) |
|---|---|---|
| SacI | 1 | 40,0 |
| StuI | 2 | 32,9, 6,9 |
| BglII | 3 | 20,8, 14,4, 4,0 |
| PvuII | 3 | 22,9, 8,3, 5,1 |
| ClaI | 4 | 21,2, 7,4, 6,1, 4,8 |
| EcoRV | 6 | 25,1, 5,9, 4,5, 3,1, 2,7, 2,1 |
| HaeIII | 4 | 25,1, 5,49, 5,0, 4,36 |
| HincII | 6 | 11,8, 5,6, 4,5, 3,8, 2,5, 1,7 |
| AvaI | 5 | 16,3, 12,5, 9,1, 4,6, 3,2 |
| HpaII | 8 | 12,6, 11,9, 10,8, 3,9, 2,4, 2,2, 1,2, 0,4 |
| EcoRI | 8 | 21,0, 12,1, 2,5, 2,1, 1,7, 0,84, 0,69, 0,41 |

**8.** Procédé de préparation d'une séquence d'ADN dans laquelle un plasmide isolé par un procédé suivant la revendication 6 ou 7 est sélectionné, et l'ADN codant pour des déterminants d'insensibilité vis-à-vis de phages, ou un ADN présentant une homologie de séquence suffisante avec cet ADN pour conférer également une insensibilité vis-à-vis de phages à des bactéries appartenant à S. lactis ou S. lactis sous-espèce diacetylactis ainsi qu'à S. cremoris à des températures allant jusqu'à 39°C est isolé.

**9.** Procédé de préparation d'un hôte bactérien, dans lequel un ou plusieurs plasmides isolés par un procédé suivant l'une quelconque des revendications 1 à 7 sont insérés dans une bactérie, l'association hôte/plasmide n'étant pas une association naturelle.

**10.** Procédé suivant la revendication 9, dans lequel l'hôte est un hôte consistant en un streptocoque lactique.

**11.** Procédé pour conférer une insensibilité vis-à-vis de phages à une bactérie utilisée pour la production du fromage, comprenant l'introduction dans la bactérie d'un plasmide isolé par un procédé suivant l'une quelconque des revendications 1 à 7, de manière à fournir une association hôte bactérien/plasmide autre qu'une association hôte bactérien/plasmide naturelle.

**12.** Procédé pour conférer une insensibilité vis-à-vis de phages à une culture de départ pour produit alimentaire comprenant des bactéries, dans lequel un plasmide isolé par un procédé suivant l'une quelconque des revendications 1 à 7 est introduit dans lesdites bactéries, de manière à fournir une association hôte bactérien/plasmide autre qu'une association hôte bactérien/plasmide naturelle.

**13.** Procédé suivant la revendication 12, dans lequel la culture de départ est choisie entre des levains pour produits laitiers, des levains pour fromages, des cultures de viande et de fermentation de viande, des cultures de fermentation de légumes et des cultures probiotiques.

**14.** Procédé suivant la revendication 13, dans lequel la culture est entreposée sous forme liquide, congelée, lyophilisée, congelée concentrée ou lyophilisée concentrée.

EP 0 246 909 B1

EP 0 246 909 B1

FIG 4 RESTRICTION MAPS OF PHAGE RESISTANCE PLASMIDS

pCI750

pCI829

FIG. 5

1 2 3 4

pCl829

EP 0 246 909 B1

42